# EUROPEAN PATENT APPLICATION

(11) **EP 3 214 182 A1**
(43) Date of publication of application: **06.09.2017**
(21) Application number: 15854844.6
(22) Date of filing: 28.10.2015
(51) Int. Cl.: C12Q 1/68, C12N 15/09, G01N 33/15, G01N 33/50

(54) **HIV PATHOLOGY MARKER AND EXAMINATION METHOD**

(30) Priority: 31.10.2014 JP 2014222314
(71) Applicant: Keio University, Tokyo 108-8345 (JP); Nikon Corporation, Minato-ku, Tokyo 108-6290 (JP)
(72) Inventor: KATO, Shingo, Tokyo 160-8582 (JP)
(74) Representative: Hoffmann Eitle
(86) International application number: PCT/JP2015/080398
(87) International publication number: WO 2016/068193

(57) **Abstract**

A technique for detecting intact provirus more simply and at a lower cost is provided. A marker showing HIV pathology, comprising near-full-length HIV provirus. A method of testing HIV pathology, comprising evaluating the level of full-length HIV provirus in HIV-infected cells. A method of testing the efficacy of an anti-HIV drug, comprising evaluating the level of near-full-length HIV provirus in HIV-infected cells. A method of screening for drugs that enable a functional cure of HIV, comprising evaluating the level of near-full-length HIV provirus in HIV-infected cells. A method of evaluating the level of near-full-length HIV provirus in HIV-infected cells, comprising detecting 3 to 7 regions scattered across the full length of HIV provirus. Also provided is a kit for evaluating the level of near-full-length HIV provirus, comprising primers capable of amplifying 3 to 7 regions scattered across the full length of HIV provirus and probes capable of detecting the nucleic acid amplified by the primers.

SEQUENCE LISTING

<110>  Keio University

<120>  HIV pathologic marker and a test method using the same

<130>  FP-201PCT

<150>  JP 2014-222314
<151>  2014-10-31

<160>  40

<170>  PatentIn version 3.5

<210>  1
<211>  20
<212>  DNA
<213>  Artificial Sequence

<220>
<223>  primer

<400>  1
atctgagcct gggagctctc                                                   20


<210>  2
<211>  21
<212>  DNA
<213>  Artificial Sequence

<220>
<223>  primer

<400>  2
taatacygac gctctcgcmc c                                                 21


<210>  3
<211>  25
<212>  DNA
<213>  Artificial Sequence

<220>
<223>  primer

<400>  3
cactagaaga aatgatgaca gcatg                                             25


<210>  4
<211>  19
<212>  DNA
<213>  Artificial Sequence

<220>
<223>  primer

<400>  4
aagctctcyk ctggtggrg                                                    19

<210>  5
<211>  22
<212>  DNA
<213>  Artificial Sequence

<220>
<223>  primer

<400>  5
aacagatggc aggtgatgat tg                                              22


<210>  6
<211>  24
<212>  DNA
<213>  Artificial Sequence

<220>
<223>  primer

<400>  6
tttcctccat tctayggagr ctcc                                            24


<210>  7
<211>  23
<212>  DNA
<213>  Artificial Sequence

<220>
<223>  primer

<400>  7
atgagggaya attggagaag tga                                             23


<210>  8
<211>  20
<212>  DNA
<213>  Artificial Sequence

<220>
<223>  primer

<400>  8
ctgcgcccat agtgcttcct                                                 20


<210>  9
<211>  23
<212>  DNA
<213>  Artificial Sequence

<220>
<223>  primer

<400>  9
gcagtagstg agggracaga tag                                             23


<210>  10
<211>  22
<212>  DNA

<213>  Artificial Sequence

<220>
<223>  primer

<400>  10
tgatccctgg ccctgktgtg ta                                                22


<210>  11
<211>  21
<212>  DNA
<213>  Artificial Sequence

<220>
<223>  primer

<400>  11
gctarctagg gaacccactg c                                                 21


<210>  12
<211>  20
<212>  DNA
<213>  Artificial Sequence

<220>
<223>  primer

<400>  12
gggcgccact gctagagatt                                                   20


<210>  13
<211>  20
<212>  DNA
<213>  Artificial Sequence

<220>
<223>  primer

<400>  13
argggctgtt ggaaatgtgg                                                   20


<210>  14
<211>  23
<212>  DNA
<213>  Artificial Sequence

<220>
<223>  primer

<400>  14
ttccctaaaa aattagcctg yct                                               23


<210>  15
<211>  22
<212>  DNA
<213>  Artificial Sequence

<220>

<223>  primer

<400>  15
gtggcaagtr gacaggatga gg                                                22


<210>  16
<211>  23
<212>  DNA
<213>  Artificial Sequence

<220>
<223>  primer

<400>  16
cctgtatgca gaccccaata tgt                                               23


<210>  17
<211>  24
<212>  DNA
<213>  Artificial Sequence

<220>
<223>  primer

<400>  17
aattgaacca ttaggantag cacc                                              24


<210>  18
<211>  22
<212>  DNA
<213>  Artificial Sequence

<220>
<223>  primer

<400>  18
cttcctgctg ctcccaagaa cc                                                22


<210>  19
<211>  25
<212>  DNA
<213>  Artificial Sequence

<220>
<223>  primer

<400>  19
cctcaggtac ctttaagrcc aatga                                             25


<210>  20
<211>  24
<212>  DNA
<213>  Artificial Sequence

<220>
<223>  primer

<400>  20

ggagtaaatt adcccttcca gtcc                                              24

<210>  21
<211>  22
<212>  DNA
<213>  Artificial Sequence

<220>
<223>  primer

<400>  21
ggctaactag ggaacccact gc                                                22


<210>  22
<211>  18
<212>  DNA
<213>  Artificial Sequence

<220>
<223>  primer

<400>  22
agcaagccga gtcctgcg                                                     18


<210>  23
<211>  20
<212>  DNA
<213>  Artificial Sequence

<220>
<223>  primer

<400>  23
gggacatcaa gcagcyatgc                                                   20


<210>  24
<211>  29
<212>  DNA
<213>  Artificial Sequence

<220>
<223>  primer

<400>  24
atccatccta tttgttcctg aaggktact                                         29


<210>  25
<211>  26
<212>  DNA
<213>  Artificial Sequence

<220>
<223>  primer

<400>  25
cctaggtgtg ahtatcmagc aggaca                                            26


<210>  26

<211>  23
<212>  DNA
<213>  Artificial Sequence

<220>
<223>  primer

<400>  26
gtcttckggg gcttgttcca tct                                               23


<210>  27
<211>  28
<212>  DNA
<213>  Artificial Sequence

<220>
<223>  primer

<400>  27
gagatatgar ggayaattgg agaagtga                                          28


<210>  28
<211>  20
<212>  DNA
<213>  Artificial Sequence

<220>
<223>  primer

<400>  28
gcgcccatag tgcttcctgc                                                   20


<210>  29
<211>  23
<212>  DNA
<213>  Artificial Sequence

<220>
<223>  primer

<400>  29
gtgggttttc cagtcarrcc tca                                               23


<210>  30
<211>  20
<212>  DNA
<213>  Artificial Sequence

<220>
<223>  primer

<400>  30
ryccctggcc ctggtgtgta                                                   20


<210>  31
<211>  22
<212>  DNA
<213>  Artificial Sequence

<220>
<223>  primer

<400>  31
gcttcaagta gtgtgtgccc gt                                                22


<210>  32
<211>  22
<212>  DNA
<213>  Artificial Sequence

<220>
<223>  primer

<400>  32
gggcgccact gctagagatt tt                                                22


<210>  33
<211>  26
<212>  DNA
<213>  Artificial Sequence

<220>
<223>  primer

<400>  33
caatgaggaa gctgcagaat gggata                                            26


<210>  34
<211>  25
<212>  DNA
<213>  Artificial Sequence

<220>
<223>  primer

<400>  34
tagttcctgc tatgtcactt cccct                                             25


<210>  35
<211>  27
<212>  DNA
<213>  Artificial Sequence

<220>
<223>  primer

<400>  35
caaggtagga tcyctacart acttggc                                           27


<210>  36
<211>  26
<212>  DNA
<213>  Artificial Sequence

<220>
<223>  primer

<400>  36
agtttcbyaa cactaggyaa aggtgg                                            26


<210>  37
<211>  29
<212>  DNA
<213>  Artificial Sequence

<220>
<223>  primer

<400>  37
gtaaaaattg aaccattagg rrtagcacc                                         29


<210>  38
<211>  21
<212>  DNA
<213>  Artificial Sequence

<220>
<223>  primer

<400>  38
gcttcctgct gctcccaara a                                                 21


<210>  39
<211>  25
<212>  DNA
<213>  Artificial Sequence

<220>
<223>  primer

<400>  39
gacaagatat ccttgayctg tggrt                                             25


<210>  40
<211>  25
<212>  DNA
<213>  Artificial Sequence

<220>
<223>  primer

<400>  40
gtgtgtagtt mtgccartca gggaa                                             25

## Description

### TECHNICAL FIELD

The present invention relates to an HIV pathology marker and a test method.

### BACKGROUND ART

Recently, treatment of HIV infections has progressed remarkably, improving the prevalence and mortality of HIV-infected individuals greatly. However, this treatment (therapy) should be continued for life; besides, the occurrence of drug resistant virus and adverse results thereof have become problems. In addition, the increasing cost of treatment that accompanies the increase in the number of infected people has become a big social problem. Under these circumstances, "a functional cure" that is a state where viral proliferation is completely inhibited and the disease does not proceed even after interruption of anti-HIV therapy has been attracting attention. Investigations into treatment methods for realizing such a state are greatly expected. For advancing these investigations, it is necessary to determine the size of viral focus (reservoir) which is difficult to reduce by anti-HIV therapy. However, an appropriate measurement method therefor has not been established yet.

The following two representative techniques have been reported as methods for quantifying the reservoir of HIV infections.
Viral outgrowth assay:
Siliciano JD, Siliciano RF. (2005) Enhanced culture assay for detection and quantitation of latently infected, resting CD4+ T-cells carrying replication competent virus in HIV-1-infected individuals. Methods Mol Biol 304: 3-15. (Non-Patent Document No. 1)
Droplet digital PCR for HIV-1 DNA:
Strain MC, Lada SM, Luong T, Rought SE, Gianella S, et al. (2013) Highly accurate measurement of HIV DNA by droplet digital PCR. PLOS ONE 8: e55943. (Non-Patent Document No. 2)

Since the above-mentioned viral outgrowth assay requires operations for culturing infectious HIV, it has a risk of infection, involves complicated operations, is time-consuming, and expensive. With respect to the above-mentioned droplet digital PCR for HIV-1 DNA, even incomplete provirus is included in quantification, so it is impossible to accurately evaluate HIV reservoirs.

### PRIOR ART LITERATURE

### Non-Patent Documents

Non-Patent Document No. 1:
   Siliciano JD, Siliciano RF. (2005) Enhanced culture assay for detection and quantitation of latently infected, resting CD4+ T-cells carrying replication competent virus in HIV-1-infected individuals. Methods Mol Biol 304: 3-15.
Non-Patent Document No. 2
   Strain MC, Lada SM, Luong T, Rought SE, Gianella S, et al. (2013) Highly accurate measurement of HIV DNA by droplet digital PCR. PLOS ONE 8: e55943.

### DISCLOSURE OF THE INVENTION

### PROBLEM FOR SOLUTION BY THE INVENTION

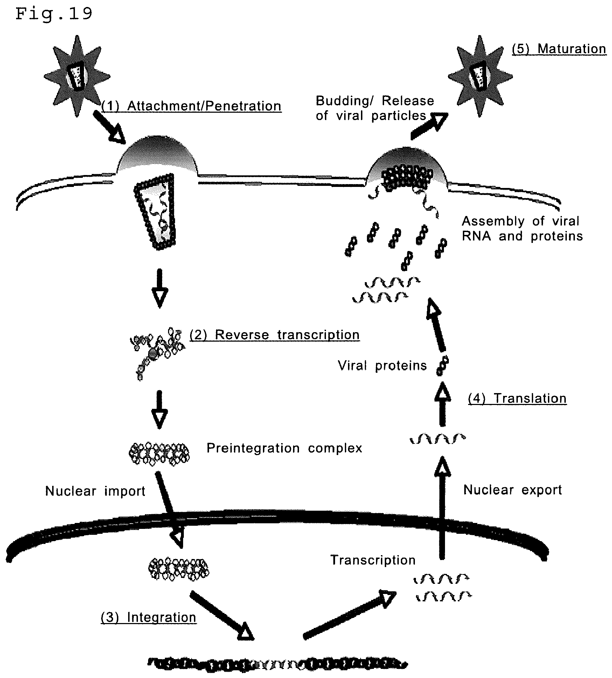

The RNA in HIV viral particles is a positive single-stranded RNA and assumes the structure of mRNA in hosts. The viral RNA is converted into a double-stranded DNA by reverse transcriptase (RT) in hosts and incorporated into the host DNA by integrase to form provirus (Fig. 19).

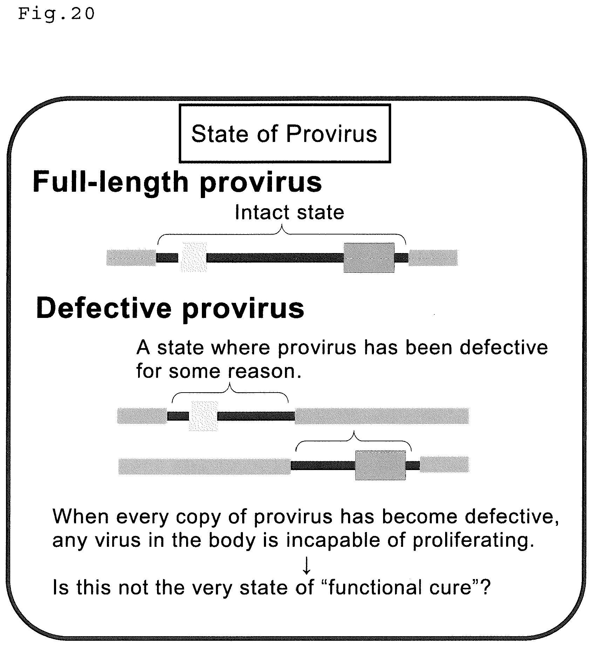

It is believed that HIV reservoir is composed of infected cells with infectious nature. In infected cells, HIV provirus exists in the state of either full-length provirus or defective provirus (provirus that has been defective for some reason). Any infected cells with infectious nature should always contain full-length provirus. When every copy of the provirus has become defective, any HIV virus in the body is incapable of proliferating, and this would be the very state called "a functional cure" (Fig. 20). However, it is difficult to directly detect full-length provirus.
Therefore, the present invention aims at providing a technique for detecting intact or untruncaed provirus more simply and at lower cost.

### MEANS TO SOLVE THE PROBLEM

As a result of intensive efforts to solve the above problems, the present inventor has established a measurement method which, in order to estimate the level of full-length provirus, quantifies a provirus that simultaneously has several sites scattered across the full length of provirus (such provirus is hereinafter called "a near-full-length provirus"). Further, using this method, the present inventor measured the levels of near-full-length provirus present in mononuclear cells in the peripheral blood of patients before and after the start of anti-HIV therapy, and examined the clinical significance of such levels. The present invention has been achieved based on these findings.

A summary of the present invention is as described below.
(1) A marker showing HIV pathology, comprising near-full-length HIV provirus.
(2) A method of testing HIV pathology, comprising evaluating the level of near-full-length HIV provirus in HIV-infected cells.
(3) The method of (2) above, wherein it is judged that a functional cure of HIV has been achieved when near-full-length HIV provirus in infected cells is evaluated as below a specific detection limit, and it is judged that a functional cure of HIV has not been achieved when near-full-length HIV provirus in infected cells is evaluated as equal to or above the specific detection limit.
(4) The method of (2) or (3) above, wherein the level of near-full-length HIV provirus in HIV-infected cells is evaluated by detecting 3 to 7 regions scattered across the full length of HIV provirus.
(5) A method of testing the efficacy of an anti-HIV drug, comprising evaluating the level of near-full-length HIV provirus in HIV-infected cells.
(6) The method of (5) above, wherein the level of near-full-length HIV provirus in HIV-infected cells is evaluated before and after anti-HIV drug administration and/or before and after a specific period during continuation of anti-HIV drug administration, and it is judged that a functional cure of HIV has been achieved or is being achieved when the copy number of near-full-length HIV provirus has decreased, and it is judged that the anti-HIV drug does not enable a functional cure of HIV when the level of near-full-length HIV provirus has not decreased.
(7) A method of screening for drugs that enable a functional cure of HIV, comprising evaluating the level of near-full-length HIV provirus in HIV-infected cells.
(8) The method of (7) above, wherein the level of near-full-length HIV provirus in HIV-infected cells is evaluated before and after administration of a drug and/or before and after a specific period during continuation of administration of the drug, and it is judged that the drug will enable a functional cure of HIV when the level of near-full-length HIV provirus has decreased, and it is judged that the drug does not enable a functional cure of HIV when the level of near-full-length HIV provirus has not decreased.
(9) A method of evaluating the level of near-full-length HIV provirus in HIV-infected cells, comprising detecting 3 to 7 regions scattered across the full length of HIV provirus.
(10) The method of any one of (1) to (9) above, comprising evaluating the level of near-full-length HIV provirus in HIV-infected cells using primers of 20 to 30 nucleotides comprising a part or the whole of any nucleotide sequence as shown in SEQ ID NOS: 1 to 40.
(11) A kit for evaluating the level of near-full-length HIV provirus, comprising primers capable of amplifying 3 to 7 regions scattered across the full length of HIV provirus and reagents capable of detecting the nucleic acid amplified by the primers.
(12) The kit of (11) above, wherein the primers capable of amplifying 3 to 7 regions scattered across the full length of HIV provirus are primers of 20 to 30 nucleotides comprising a part or the whole of any nucleotide sequence as shown in SEQ ID NOS: 1 to 40.

### EFFECT OF THE INVENTION

According to the present invention, it has become possible to estimate the level of HIV's near-full-length provirus. Based on such estimation, it becomes possible to judge whether a functional cure of HIV infections has been achieved. Consequently, simplification of anti-HIV therapy can be achieved. Although anti-HIV therapy has made a remarkable progress recently, the problems of adverse effects and drug resistance have not yet been resolved. Besides, once started, anti-HIV therapy should be continued for life. If the therapy can be simplified or judgement can be made for its interruption or termination according to the present invention, this will not only improve the quality of life (QOL) of patients under the therapy dramatically but it also leads to considerable reduction in medical expenses.

The present invention encompasses the contents disclosed in the specification and/or drawings of Japanese Patent Application No. 2014-222314 based on which the present patent application claims priority.

### BRIEF DESCRIPTION OF THE DRAWINGS

Fig. 1 shows the method of judgement for positive reactions in Real-Time PCR using SYBR Green.
Fig. 2 shows the time course of CD4 levels and virus levels in 7 blood samples of a patient over 9 years after start of anti-HIV therapy
Fig. 3 shows the structure of HIV-1 plasmid (sample: pREjo.c).
Fig. 4 shows the structure of HIV-1 plasmid (sample: pCH077.t).
Fig. 5 shows the structure of HIV-1 plasmid (sample: pNL43).
Fig. 6 shows the structure of HIV-1 plasmid pNL43 after preparation operations (the first experiment).
Fig. 7 shows the structure of HIV-1 plasmid pNL43 after preparation operations (the second experiment).
Fig. 8 shows the structure of HIV-1 plasmid pNL43 after preparation operations (the third experiment).
Fig. 9 shows the structure of provirus in the peripheral blood mononuclear cells (PBMCs) of an HIV-1-infected individual (sample: C97SP369).
Fig. 10 shows the structure of provirus in the PBMCs of an HIV-1-infected individual (sample: C119SP429).
Fig. 11 shows the structure of provirus in the PBMCs of an HIV-1-infected individual (sample: C125SP447).
Fig. 12 shows the structure of provirus in the PBMCs of an HIV-1-infected individual (sample: C369SP1131).
Fig. 13 shows the structure of provirus in the PBMCs of an HIV-1-infected individual (sample: C370SP1138).
Fig. 14 shows the structure of provirus in the PBMCs of an HIV-1-infected individual (sample: C272SP1117).
Fig. 15 shows the structure of provirus in the PBMCs of an HIV-1-infected individual (sample: C95SP1162).
Fig. 16 shows the structure of provirus in the PBMCs of an HIV-1-infected individual (sample: C232SP1163).
Fig. 17 shows the structure of provirus in the PBMCs of an HIV-1-infected individual (sample: C252SP1164).
Fig. 18 shows the structure of provirus in the PBMCs of an HIV-1-infected individual (sample: C193SP1174).
Fig. 19 is a schematic diagram showing how HIV virus infects a host and amplifies.
Fig. 20 shows two possible states of HIV provirus.
Fig. 21 shows the positions of primers designed for amplifying 5 sites in preserved regions in HIV genome.
Fig. 22 shows the structure of provirus in the PBMCs of an HIV-1-infected individual (follow-up study for 9 years after start of anti-HIV therapy) (sample: C53SP271).
Fig. 23 shows the structure of provirus in the PBMCs of an HIV-1-infected individual (follow-up study for 9 years after start of anti-HIV therapy) (sample: C53SP278).
Fig. 24 shows the structure of provirus in the PBMCs of an HIV-1-infected individual (follow-up study for 9 years after start of anti-HIV therapy) (sample: C53SP281).
Fig. 25 shows the structure of provirus in the PBMCs of an HIV-1-infected individual (follow-up study for 9 years after start of anti-HIV therapy) (sample: C53SP294).
Fig. 26 shows the structure of provirus in the PBMCs of an HIV-1-infected individual (follow-up study for 9 years after start of anti-HIV therapy) (sample: C53SP313).
Fig. 27 shows the structure of provirus in the PBMCs of an HIV-1-infected individual (follow-up study for 9 years after start of anti-HIV therapy) (sample: C53SP679).
Fig. 28 shows the structure of provirus in the PBMCs of an HIV-1-infected individual (follow-up study for 9 years after start of anti-HIV therapy) (sample: C53SP1028).
Fig. 29 shows the level of total provirus and the level of near-full-length provirus in the PBMCs of an HIV-infected individual (C53) for 9 years after start of anti-HIV therapy.
Fig. 30 shows the results of agarose gel electrophoresis of multiplex nested PCR products.
Fig. 31 shows the results of agarose gel electrophoresis of individual second-round PCR products.

### DESCRIPTION OF EMBODIMENTS

Hereinbelow, the present invention will be described in more detail.

The present invention provides a method of testing HIV pathology, comprising evaluating the level of near-full-length HIV provirus in HIV-infected cells.

The term "HIV" used herein refers to a virus that infects human immune cells, destroy those cells and eventually causes acquired immune deficiency syndrome (AIDS). Taxonomically, HIV belongs to the genus *Lentivirus* (single-stranded, positive-sense, enveloped RNA viruses) in the family *Retroviridae.* HIV occurs in two types: HIV-1 and HIV-2. In the present invention, HIV may be either HIV-1 or HIV-2.

HIV-infected cells may include those infected cells which are contained in blood, lymph fluid, spinal fluid, semen, lymph nodes, rectum, breast milk and so forth from subjects such as infected individuals or patients whose HIV infection has been confirmed or patients who are receiving anti-HIV therapy. Specific examples include, but are not limited to, peripheral blood mononuclear cells, helper T-cells, memory T-cells, cytotoxic T-cells, monocytes, macrophages, dendritic cells and glial cells.

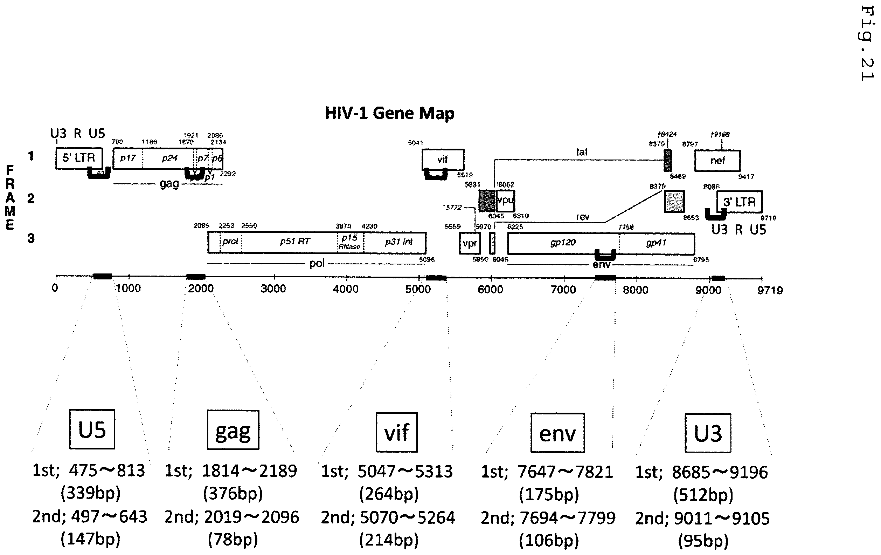

The term "near-full-length HIV provirus" used herein refers to a provirus that simultaneously has several sites scattered across the full length of HIV provirus. The near-full-length HIV provirus may be any provirus that simultaneously has 3 to 7 sites, preferably 5 to 7 sites, scattered across the full length. HIV whose RNA has a total length of about 9,000 bp has 10 genes (*gag, pro, pol, vif, vpr, vpu, tat, rev, env* and *nef*) and two LTRs (Long Terminal Repeats) at both ends (Fig. 21). The 3 to 7 scattered sites may be located in any of the regions selected from the above-mentioned 10 genes, 5' LTR and 3' LTR. Preferred sites are where mutations will rarely occur.

Evaluation of the level of near-full-length HIV provirus in HIV-infected cells may be carried out as described below.
1. Several sites scattered across the full length of HIV provirus are amplified simultaneously.

The locations and the number of sites to be amplified may be such that one can estimate whether full-length provirus is in or absent from infected cells.

The sites to be amplified may be such that mutations will rarely occur. In the case of HIV-1 provirus, examples of preferable sites include, but are not limited to, the following genetic sites: 475-813 of 5' LTR, 1814-2189 of *gag,* 5047-5313 of vif, 7647-7821 of *env,* 8685-9196 of 3' LTR, 1231-1423 of *gag,* 2598-2774 of *pol,* 4026-4223 of *pol,* and 6413-6609 of *env.*

Genetic sequence information on HIV-1 provirus is available from HIV Databases (http://www.hiv.lanl.gov/content/index).

The number of sites to be amplified is appropriately 3 to 7, preferably 5 to 7, and more preferably 5. The length of the site to be amplified is appropriately 100-2000 bp, preferably 160-550 bp.

Amplification may be carried out by multiplex PCR, but this is not the sole example and other methods such as long PCR or recombinant PCR may also be used.

As PCR primers, examples include, but are not limited to, primers of 20 to 30 nucleotides comprising a part or the whole of any nucleotide sequence as shown in SEQ ID NOS: 1-10 and 21-30. The primer length may suffice to be 20 to 30 nucleotides, preferably 20 to 25 nucleotides. Nucleotides composing the primers may be any of the following: DNA, RNA, chimeric molecules of DNA and RNA, derivatives thereof, modified forms thereof, etc.

In carrying out the amplification, positive control and/or negative control may also be provided. As positive control, pREJO.c, pCH077.t, pNL43, etc. may be used. As negative control, TE, water, PolyA, DNA from non-infected individuals, etc. may be used.

To ensure that about 20-40% of PCR reactions will give a positive result, samples as target of measurement that contain infected cells are preferably subjected to limiting dilution in advance to prepare a solution containing about 0.2-0.5 copies of HIV provirus DNA per reaction.

As a multiplex PCR device, GeneAmp PCR system 9700 (Applied Biosystems) may be used. Thermal conditions may be as follows: reaction at 94°C for 2 min, followed by 30 cycles of 3-step reaction at 94°C for 5 s, at 60°C for 10 s and at 72°C for 30 s. However, these conditions may be altered appropriately as long as the experiment can be reproduced. The present invention carried out under such altered conditions is also included within the scope of the present invention.

After completion of the PCR reaction, EDTA may be added to terminate the reaction. When long-term preservation is intended, the reaction solution is stored in a freezer (-20°C or below).
2. Amplified products from 1 above are individually amplified for each site.

The amplified products from 1 above are diluted to an appropriate ratio. Then, simultaneous or separate amplification is performed for each site.

Amplification may be performed by Real-Time PCR, but this is not the sole example and other amplification method may also be used, such as a conventional PCR followed by gel electrophoresis.

As PCR primers, examples include, but are not limited to, primers of 20 to 30 nucleotides comprising a part or the whole of any nucleotide sequence as shown in SEQ ID NOS: 11-20 and 31-40. The primer length may suffice to be 20 to 30 nucleotides, preferably 20 to 25 nucleotides. Nucleotides composing the primers may be any of the following: DNA, RNA, chimeric molecules of DNA and RNA, derivatives thereof, modified forms thereof, and so forth.

The amplification reaction of 1 and the amplification reaction of 2 described above may suffice to be a nested PCR. PCR primers used in the amplification reaction of 2 may suffice to be such that either side of their positions is set inward of the target site of PCR primers used in the amplification reaction of 1. Fig. 21 shows the concept of designing the primers used in the first-round PCR and the primers used in the second-round PCR, as performed in Examples to be described later.

The length of the site to be amplified in the amplification of 1 is appropriately 60-1800 bp, preferably 150-500 bp.

The length of the site to be amplified in the amplification of 2 is appropriately 60-1800 bp, preferably 60-250 bp.

As a Real-Time PCR device, ABI Step One plus (Applied Biosystems) may be used. Thermal conditions may be as follows: reaction at 95°C for 20 s, 50 subsequent cycles of 3-step reaction at 95°C for 1 s, at 60°C for 10 s and at 72°C for 20 s, followed by melt curve stage at 95°C for 15 s, at 60°C for 1 min and at 95°C for 15 s. However, these conditions may be altered appropriately as long as the experiment can be reproduced. The present invention carried out under such altered conditions is also included within the scope of the present invention.

For detection of the PCR amplified products, reagents such as DNA-binding intercalators (e.g., SYBR Green) may be used. Although fluorescence-labeled probes may be used as detection reagents, it is possible that no fluorescence is emitted if even a single mutation is included in the PCR amplified product. On the other hand, a DNA-binding intercalator such as SYBR Green is advantageous since it emits fluorescence even in the presence of a mutation. SYBR Green is a DNA-binding intercalator which binds to the double-stranded DNA synthesized by PCR and emits fluorescence upon irradiation with excitation light. It is possible to monitor the generation of PCR amplified products by measuring the fluorescence intensity during PCR reaction. Further, it is possible to calculate Tm values (melting temperatures) by performing melt curve analysis after PCR reaction, and from these values, it is possible to check if PCR amplified products of interest have been obtained.

As a fluorescence-labeled probe, TaqMan^{™} MGB probes, for example, may be used which are modified with the fluorophore FAM at 5' end and an NFQ (Non-Fluorescent Quencher) and the Tm enhancer MGB (Minor Groove Binder) at 3' end. The length of probes may be 10 to 40 nucleotides, preferably 20 to 30 nucleotides. Nucleotides composing the probes may be any of the following: DNA, RNA, chimeric molecules of DNA and RNA, derivatives thereof, modified forms thereof, and so forth.
3. Based on Ct values and melt curves, the results of 2 above are judged whether they are positive or negative.

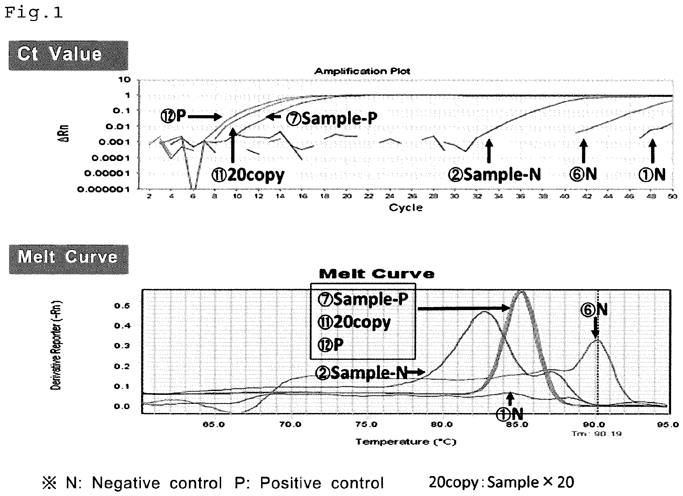

A reaction can be judged to be positive on the condition that the following three requirements are satisfied simultaneously (an example is given in Fig. 1):
(i) The Ct value obtained in the amplification reaction 2 (e.g., Real-Time PCR) is closer to that of positive control reaction than that of negative control reaction;
(ii) The melting temperature obtained from the melt curve of the amplification reaction 2 is within 0.5°C above or below the melting temperature of positive control; and
(iii) The half-width of melt curve is substantially equal to that of positive control.
Quantification of near-full-length provirus is carried out as described below. Briefly, the frequency of reactions in which PCR was not positive at any of the 5 sites in terminal dilution is written as P(0). This is substituted in Poisson's distribution formula, m=-1n(P(0)) to thereby determine the copy number (m) of near-full-length provirus contained in one reaction. When the resultant copy number is multiplied by the dilution ratio of provirus DNA solution, the initial concentration of near-full-length provirus DNA can be obtained. Quantification of defective provirus is carried out in the same manner as described above, using the frequency of reactions in which PCR was not negative at even one of the 5 sites in terminal dilution.

When near-full-length HIV provirus in infected cells is found to be less than a specific detection limit by the above-described evaluation, it can be judged that a functional cure of HIV has been achieved. When near-full-length HIV provirus in infected cells is evaluated to be equal to or above the specific detection limit, it can be judged that a functional cure of HIV has not been achieved. For example, in the method disclosed in Examples described later, the detection limit of near-full-length provirus is one copy per µg of cellular DNA. One µg of cellular DNA is the amount contained in about 140,000 lymphocytes. That is, when one or more infected cells harboring near-full-length HIV provirus are present in about 140,000 lymphocytes, the near-full length provirus can be detected in 1 µg of cellular DNA. The detection limit for making judgment about functional cure is to be determined in the future by clinical studies of functional cure using the measurement method of the present invention.

By evaluating the level of near-full-length HIV provirus in infected cells, it is possible to judge whether or not a functional cure of HIV infections has been achieved. Therefore, the present invention proves that near-full-length HIV provirus can serve as a marker showing HIV pathology.

By evaluating the level of near-full-length HIV provirus in HIV-infected cells, it is possible to test the efficacy of an anti-HIV drug. Therefore, the present invention provides a method of testing the efficacy of an anti-HIV drug, comprising evaluating the level of near-full-length HIV provirus in HIV-infected cells.

For example, the level of near-full-length HIV provirus in HIV-infected cells is evaluated before and after anti-HIV drug administration and/or before and after a specific period during continuation of anti-HIV drug administration, and it can be judged that a functional cure of HIV has been achieved or is being achieved when the copy number of near-full-length HIV provirus has decreased, and it can be judged that the anti-HIV drug does not enable a functional cure of HIV when the level of near-full-length HIV provirus has not decreased.

Further, by evaluating the level of near-full-length HIV provirus in HIV-infected cells, it is possible to screen for drugs that enable a functional cure of HIV. Therefore, the present invention provides a method of screening for drugs that enable a functional cure of HIV, comprising evaluating the level of near-full-length HIV provirus in HIV-infected cells.

For example, the level of near-full-length HIV provirus in HIV-infected cells is evaluated before and after administration of a drug and/or before and after a specific period during continuation of administration of the drug, and it can be judged that the drug will enable a functional cure of HIV when the level of near-full-length HIV provirus has decreased, and it can be judged that the drug does not enable a functional cure of HIV when the level of near-full-length HIV provirus has not decreased.

Further, the present invention also provides a method of evaluating the level of near-full-length HIV provirus in HIV-infected cells by detecting 3 to 7 regions scattered across the full length of HIV provirus. This evaluation method is as described above.

Sill further, the present invention provides a kit for evaluating the level of near-full-length HIV provirus, comprising primers capable of amplifying 3 to 7 regions scattered across the full length of HIV provirus and reagents capable of detecting the nucleic acid amplified by the primers (such as fluorescence-labeled probes, intercalators, and so forth). Near-full-length HIV provirus, the level of near-full-length HIV provirus, evaluation of the level of near-full-length HIV provirus, primers capable of amplifying 3 to 7 regions scattered across the full length of HIV provirus, and reagents capable of detecting the nucleic acid amplified by those primers are as described above. The kit may also contain a set of other reagents necessary for evaluating the level of near-full-length HIV provirus, as well as a buffer, an instruction manual, software for evaluation, and so on. The instruction manual may contain not only an explanation of how to use the kit but also a calibration curve, and criteria for predicting therapeutic effect on HIV-infected individuals, progress of pathology, timing for interruption of therapy, and so forth from the result of evaluation of near-full-length HIV provirus, as well as necessary precautions.

### EXAMPLES

Hereinbelow, the present invention will be described in more detail with reference to the following Examples. However, the present invention is not limited to these Examples.

### [Example 1]

### Experimental Methods

### (1) Method for quantifying near-full-length provirus (Tables 1 and 2, Fig. 1)

Peripheral blood mononuclear cells were prepared from blood using Ficoll-Paque (GE Health Care). DNA was extracted therefrom using DNA Blood Kit (Qiagen).

In the first-round of multiplex PCR, to ensure that about 30% of PCR reactions would give a positive result based on the Poisson probability distribution, 20 µl of TE containing about 0.4 copies of HIV-1 provirus DNA was prepared per reaction and added to 30 µl of a premix solution (whose composition is shown in Table 2) containing PCR primers (whose sequences are shown in Table 1) for 5 sites scattered across the full length of provirus. As positive control, pNL43 was used. As negative control, TE was used. As a PCR device, GeneAmp PCR system 9700 was used. PCR thermal conditions were as follows: reaction at 94°C for 2 min, followed by 30 cycles of 3-step reaction at 94°C for 5 s, at 60°C for 10 s and at 72°C for 30 s. After completion of the reaction, 1 µl of 250 nM EDTA was added per reaction to terminate the reaction. In the second round of Real-Time PCR, the first-round multiplex PCR product was diluted 100-fold and to 5 µl of the dilution, 15 µl of a premix solution containing primers whose nucleotide sequences are shown in Table 1 (the composition of the premix solution is shown in Table 2) was added. As a Real-Time PCR device, ABI Step One plus was used. Thermal conditions of Real-Time PCR were as follows: reaction at 95°C for 20 s, 50 subsequent cycles of 3-step reaction at 95°C for 1 s, at 60°C for 10 s and at 72°C for 20 s, followed by melt curve stage at 95°C for 15 s, at 60°C for 1 min and at 95°C for 15 s.

A reaction was judged to be positive on the condition that the following three requirements were satisfied simultaneously (Fig. 1):
(i) The Ct value obtained in the second round of Real-Time PCR is closer to that of positive control reaction than that of negative control reaction;
(ii) The melting temperature obtained from the melt curve is within 0.5°C below or above the melting temperature of positive control; and
(iii) The half-width of melt curve is substantially equal to that of positive control.

Quantification of near-full-length provirus was carried out as described below. Briefly, the frequency of reactions in which the result of PCR was positive at all 5 sites in terminal dilution was substituted in the probability density formula of Poisson distribution.

### (2) Accuracy of the quantified values

For each of the HIV-1 subtype B plasmids (pREJO.c, pCH077.t and pNL43, all being obtained from NIH) that had been linearized with restriction enzymes, accuracy of the quantified value was examined. As restriction enzymes, SalI was used for pREJO.c, Not I for pCH077.t, and Nco I for pNL43 (all from New England BioLabs). Plasmid concentrations were determined from absorbance at 260 nm using 1 OD = 50 mg/ml.

### (3) DNA damage at DNA extraction stages

Peripheral blood mononuclear cells prepared from the blood of non-HIV-1-infected individual were mixed with HIV-1 subtype B plasmid (pNL43) that had been linearized with restriction enzyme Nco I. From the resultant mixture, DNA was extracted with DNA Blood Kit and subjected to testing for damage.

### (4) Levels of near-full-length provirus before and after the start of anti-HIV therapy

Blood samples from treatment-naive patients (5 cases) and patients from whom no virus had been detected for more than 3 years after start of anti-HIV therapy (5 cases) were used for this testing.

### (5) Follow-up study for 9 years after start of anti-HIV therapy

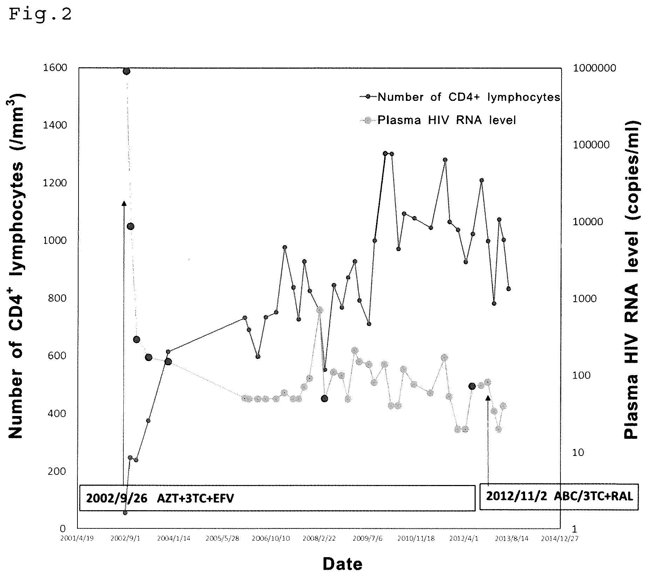

This study was carried out using 7 blood samples of a patient over 9 years after the start of anti-HIV therapy (Fig. 2). Measurement of the level of plasma HIV RNA was performed with COBAS^{™} TaqMan^{™} HIV-1 "Auto" (as entrusted to BML Inc. (Head Office: 5-21-3 Sendagaya, Shibuya-ku, Tokyo 151-0051; Research Center: 1361-1 Matoba, Kawagoe City, Saitama Pref. 350-1101)). CD4 positive lymphocytes were measured with a flow cytometer (as entrusted to the Central Clinical Laboratory, Keio University Hospital).

### Results

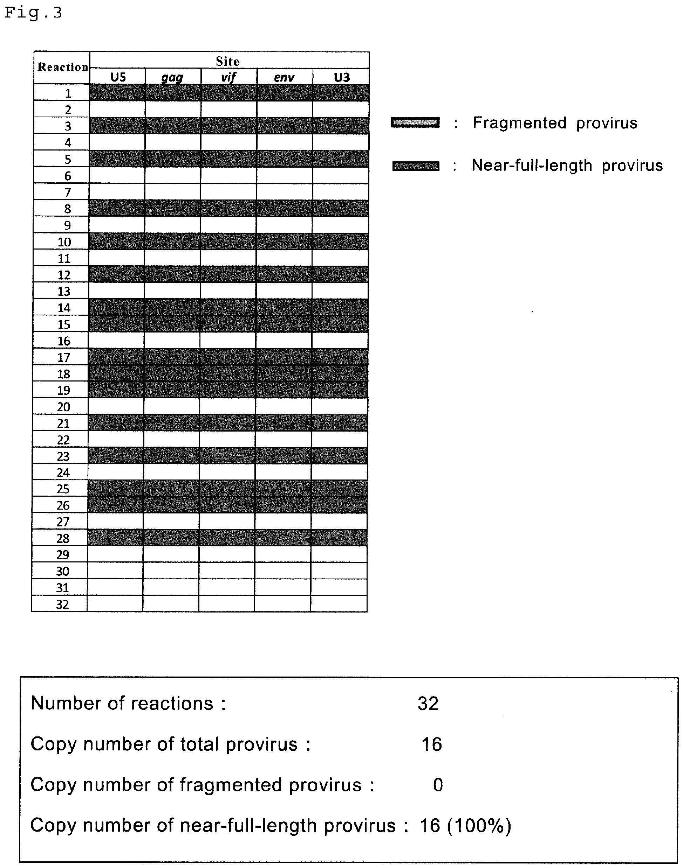

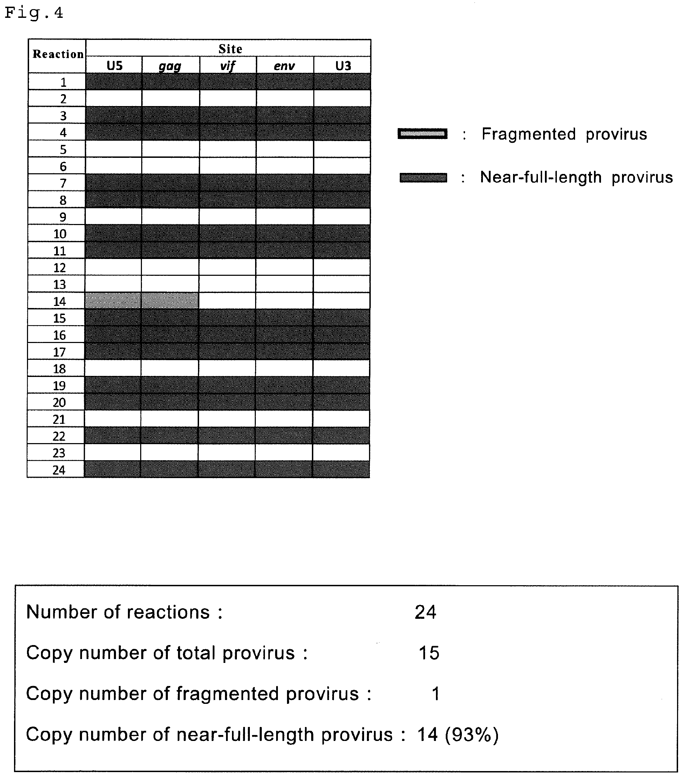

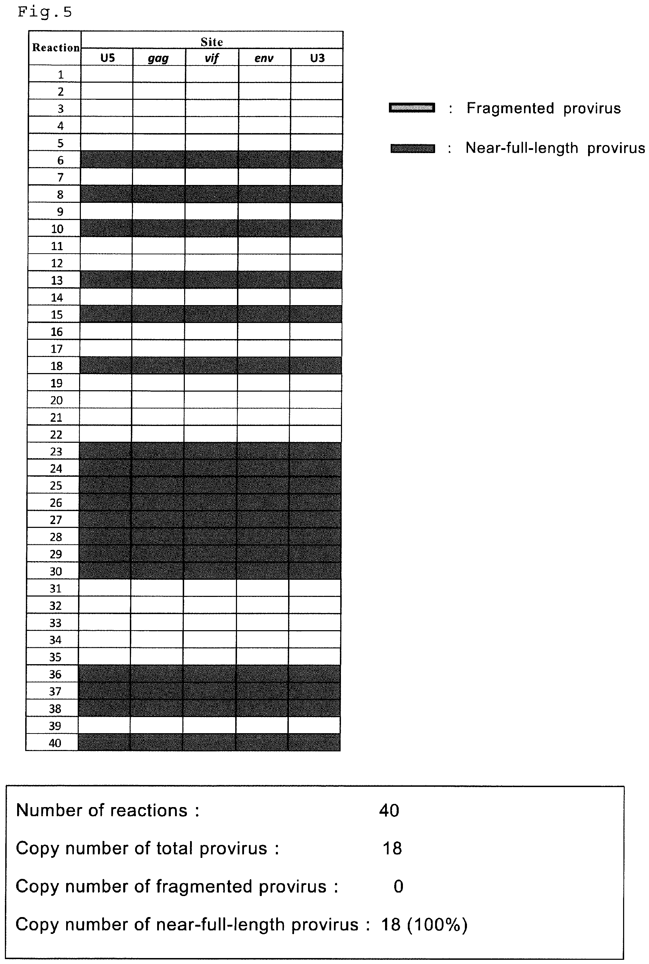

### (1) Accuracy of the quantified values (Figs. 3 to 5)

The ratio of near-full-length provirus to total provirus was 16/16 in pREJO.c, 14/15 in pCH077.t and 18/18 in pNL43, the average being 98%. These results demonstrate that it is possible to quantify near-full-length provirus by the test method of the present invention (Table 3).

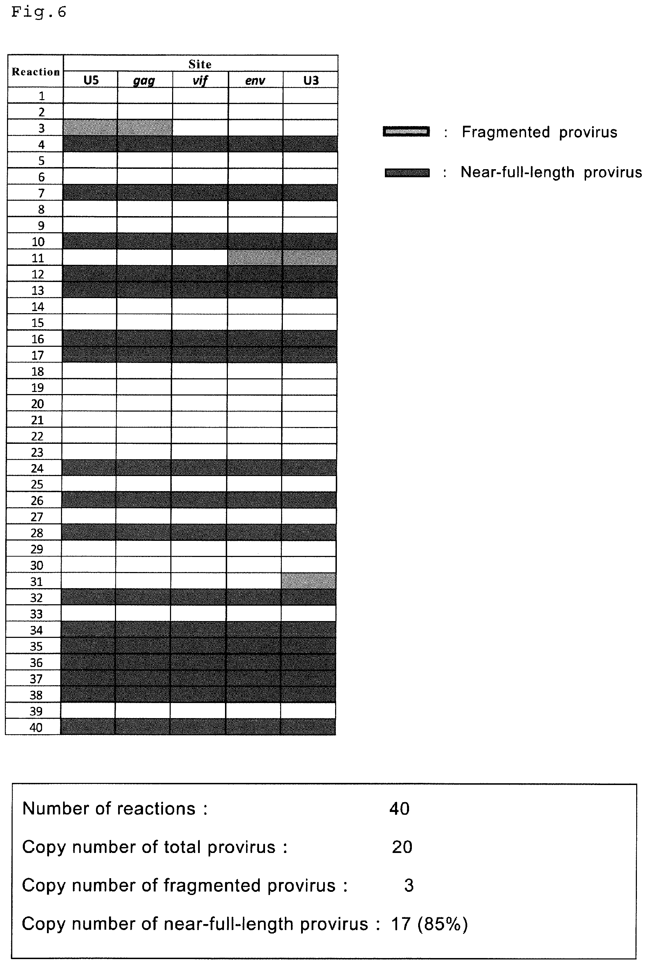

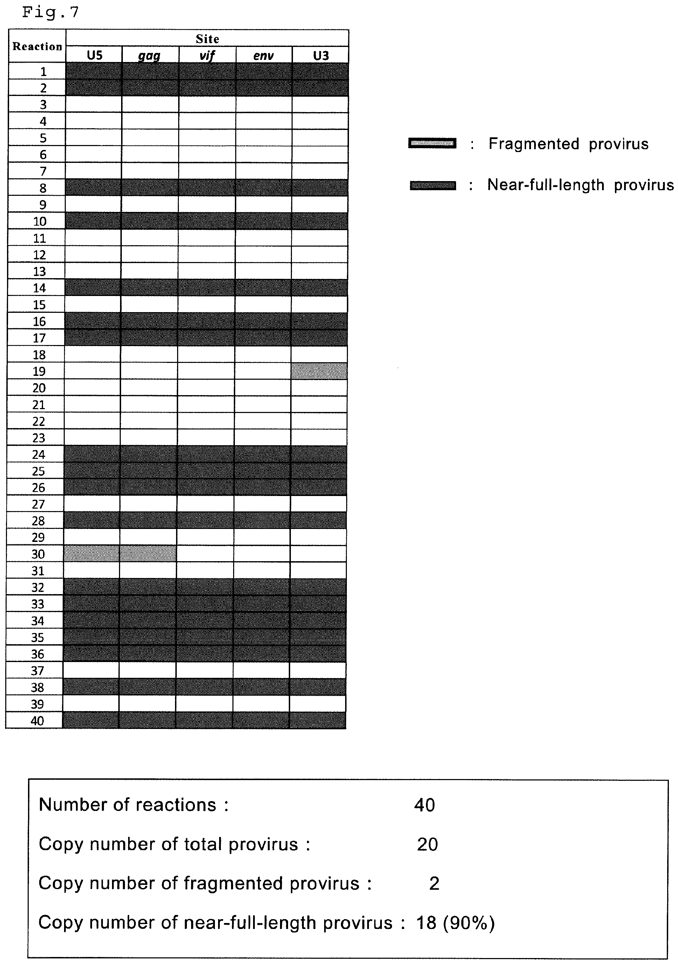

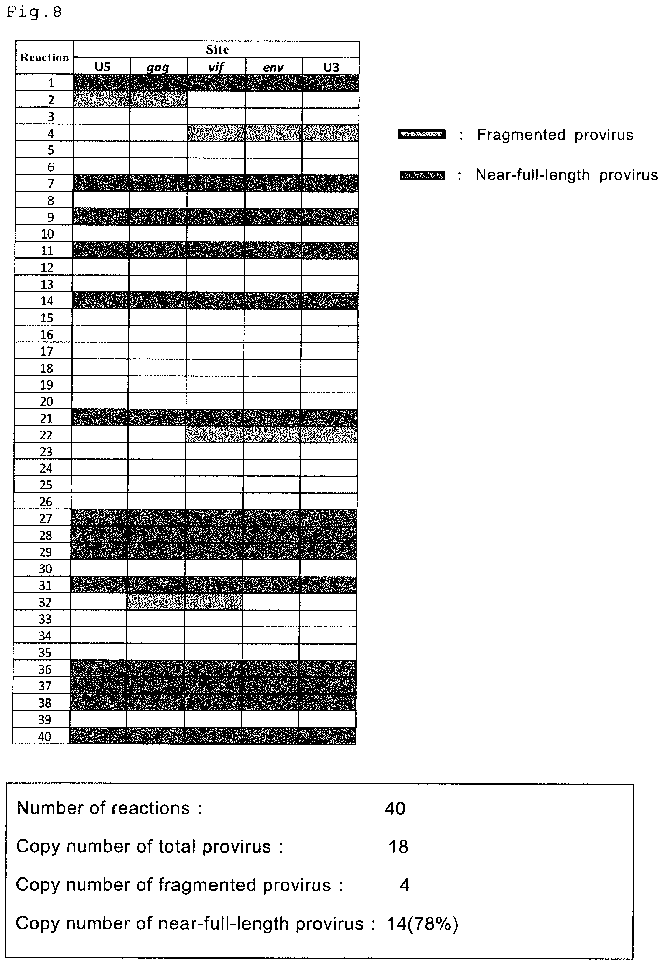

### (2) DNA damage at DNA extraction stages (Figs. 6 to 8)

The ratio of near-full-length provirus to total provirus was 17/20 in the first experiment, 18/20 in the second experiment and 14/18 in the third experiment, the average being 88%. Substantial damage to DNA by extraction operations was not recognized (Table 4).

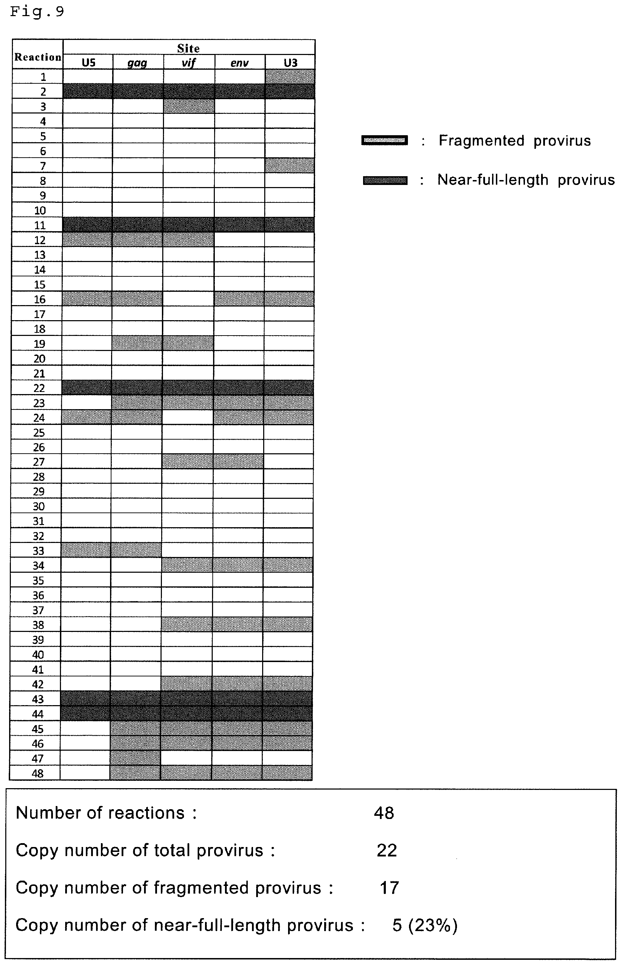

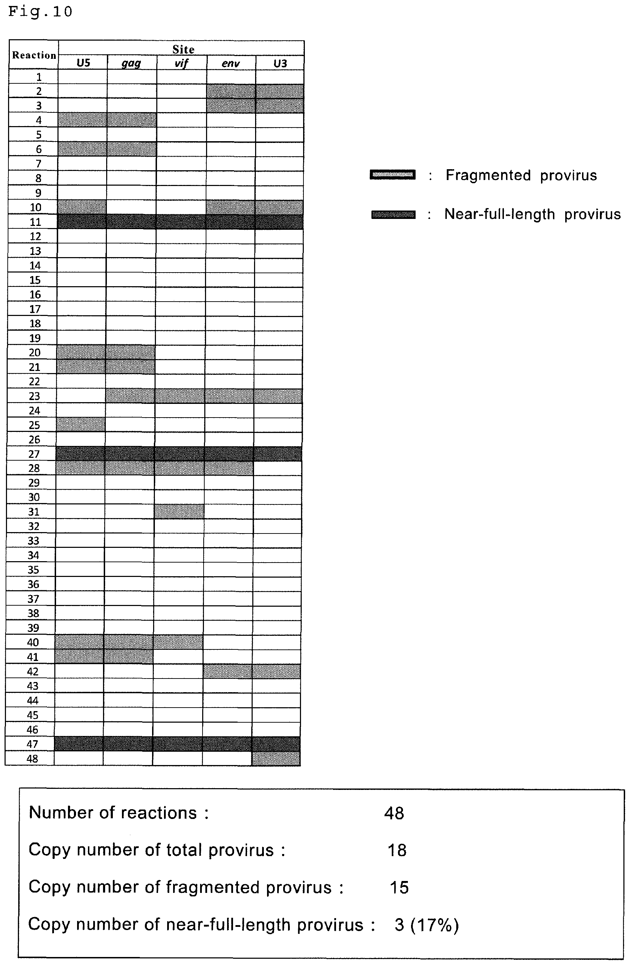

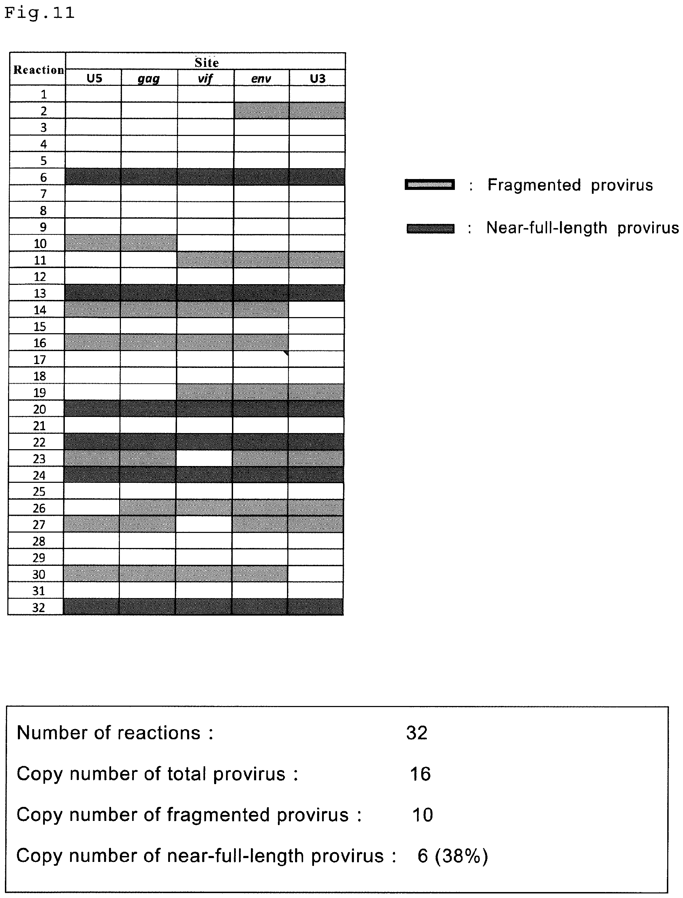

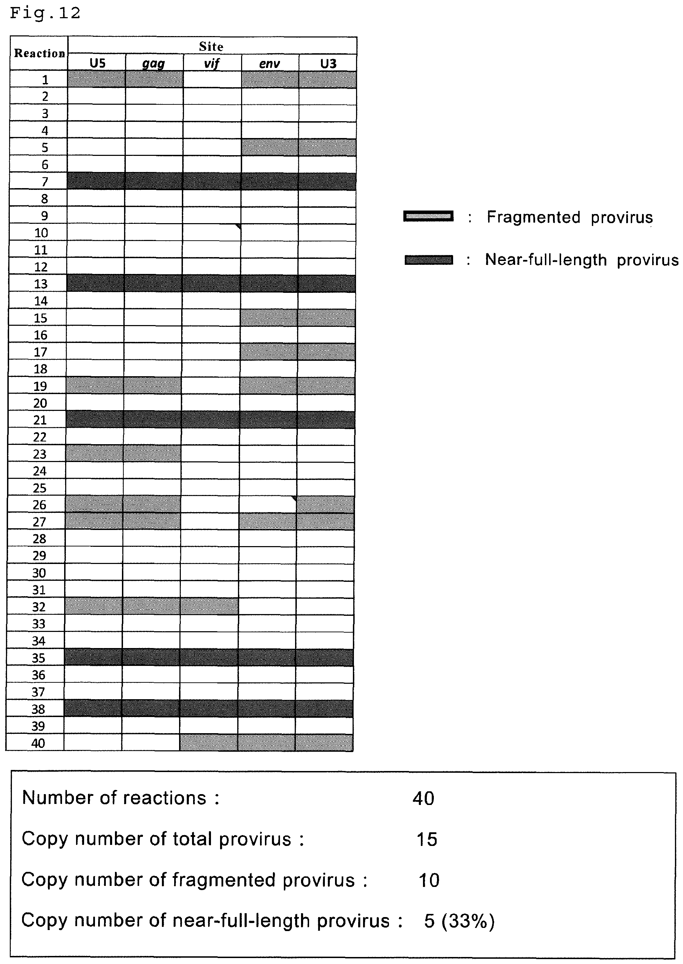

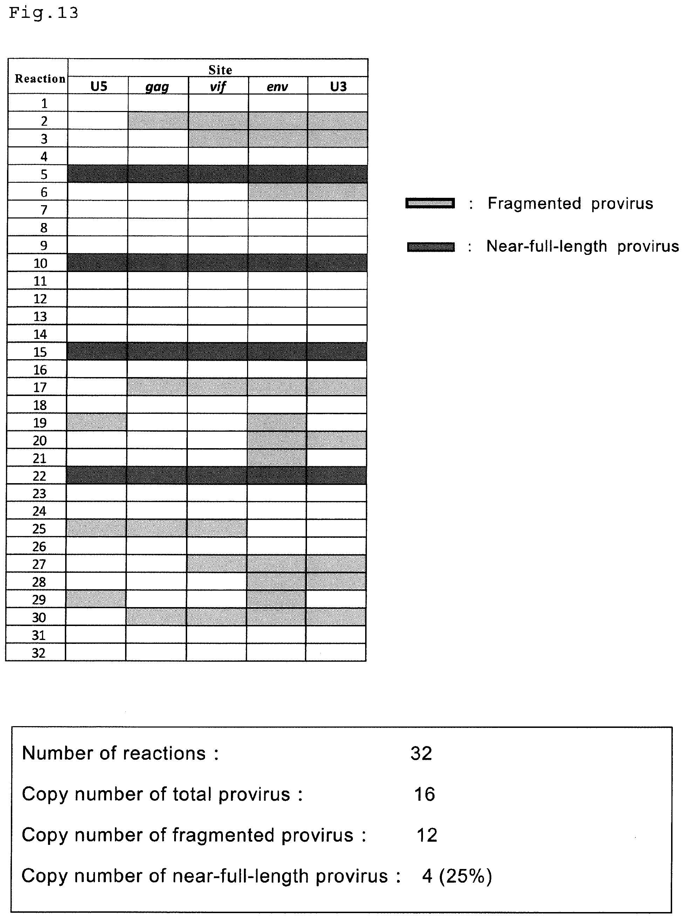

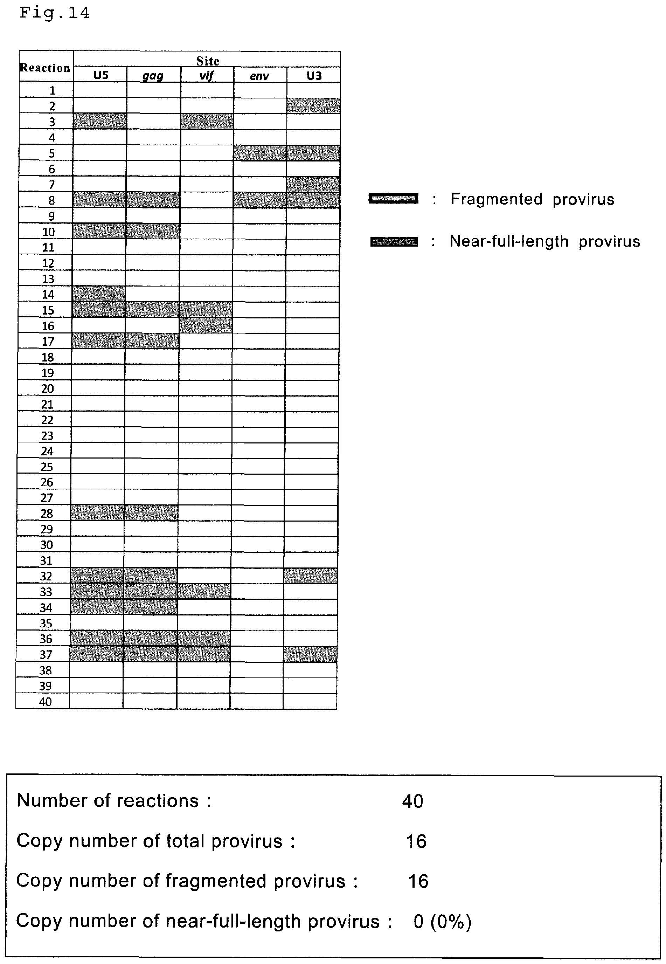

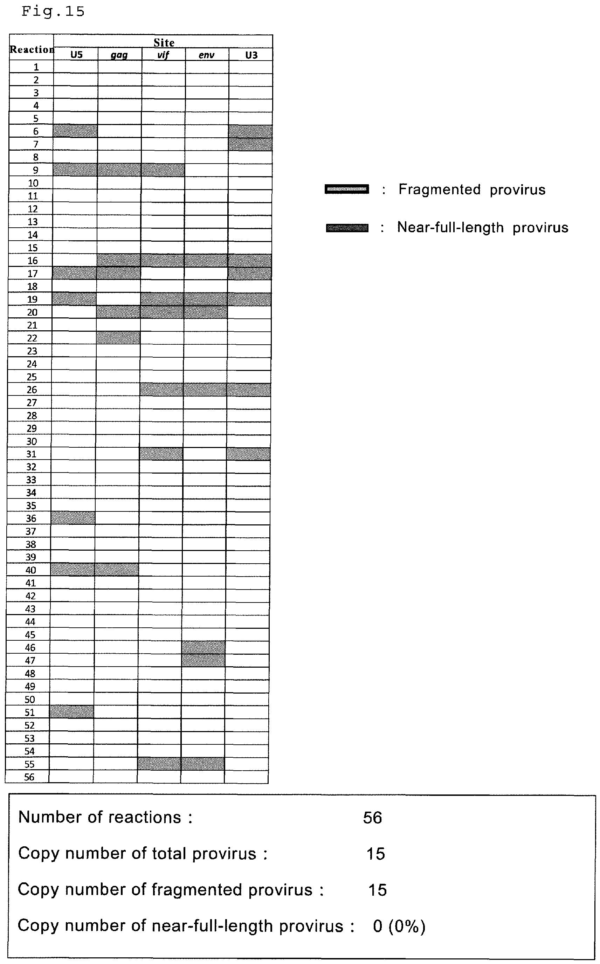

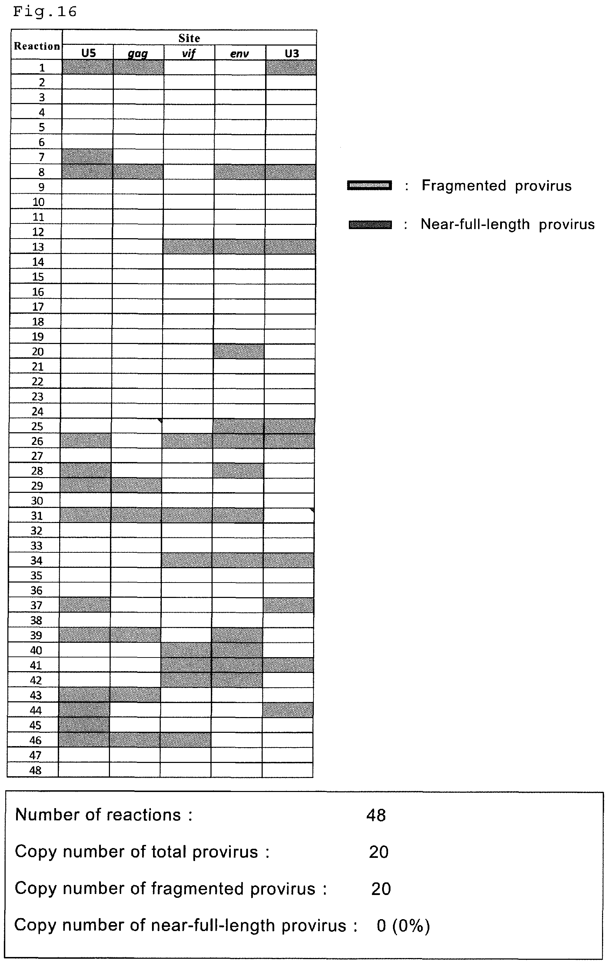

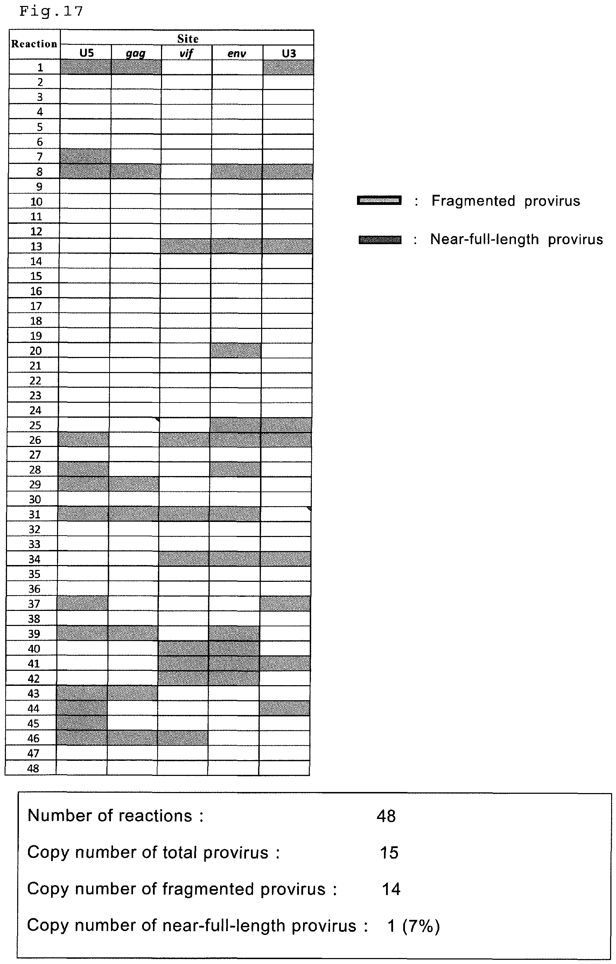

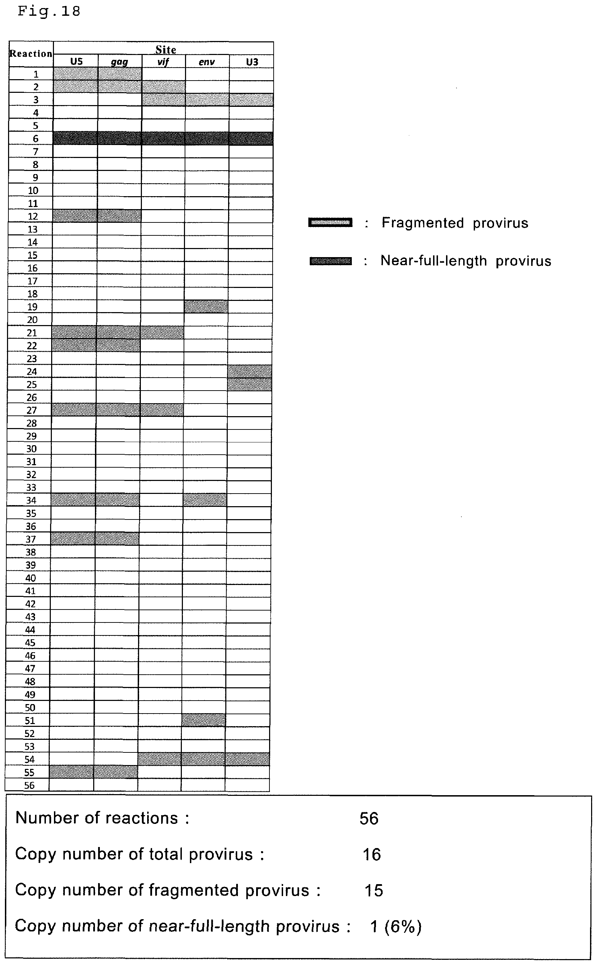

### (3) Levels of near-full-length provirus before and after the start of anti-HIV therapy (Figs. 9 to 18)

The ratio of near-full-length provirus to total provirus was 26% on average before therapy and 2% on average after therapy (Welch's t test, P=0.003). Statistically, these results were extremely significant (Table 5).

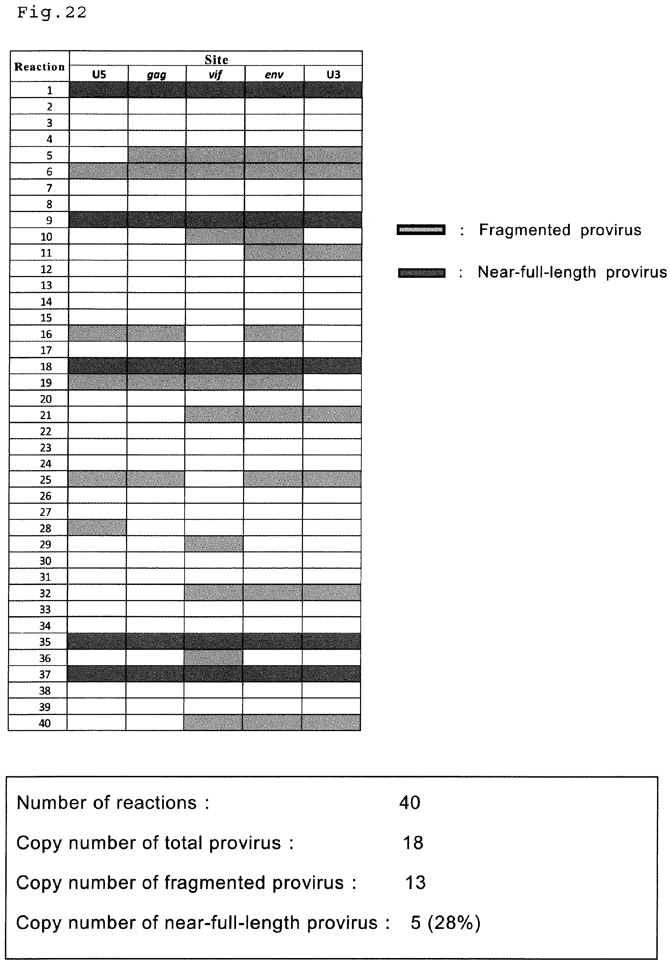

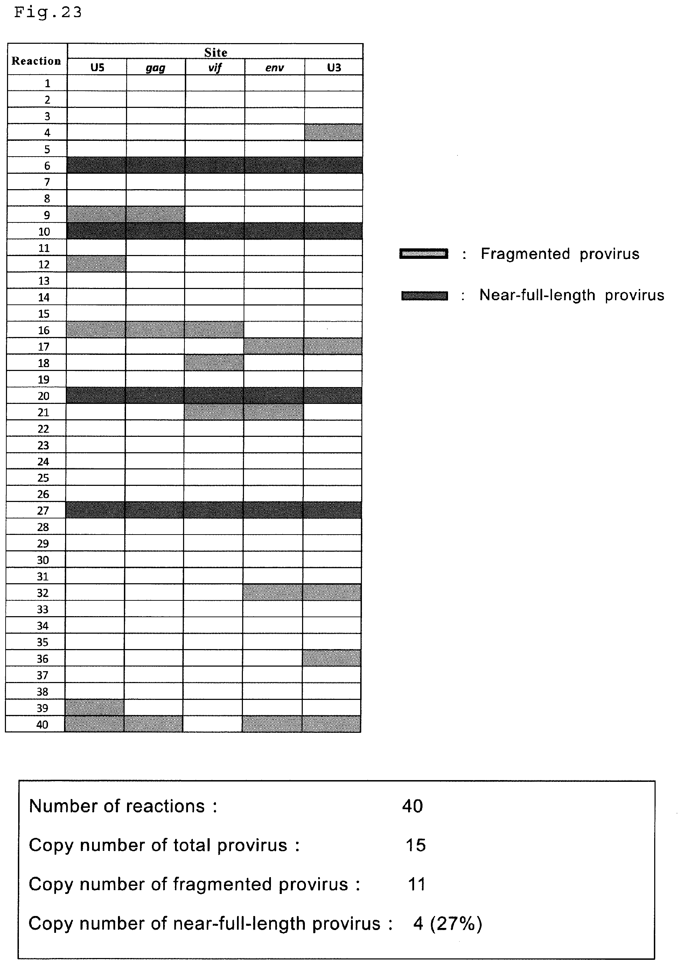

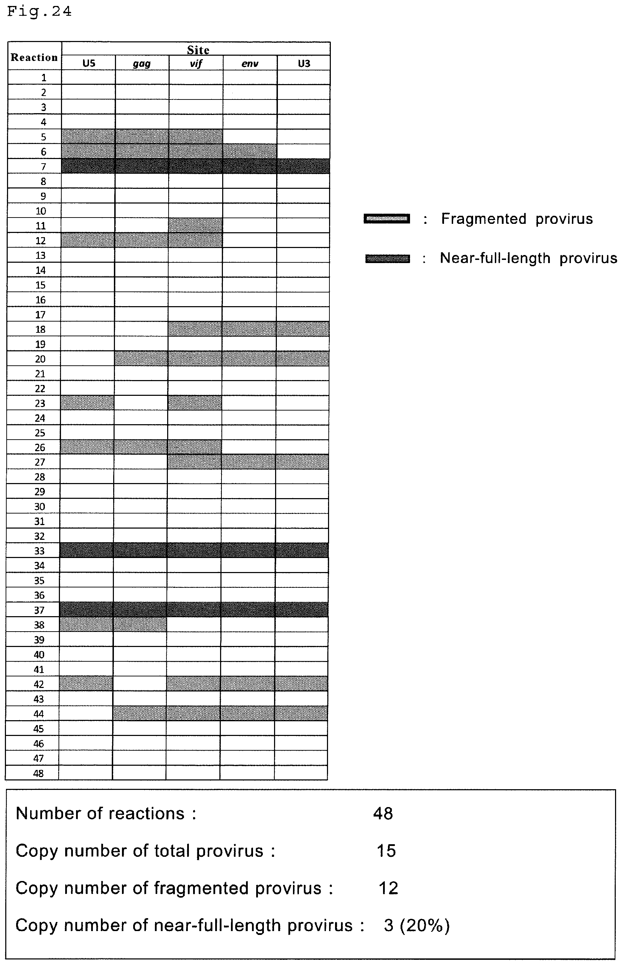

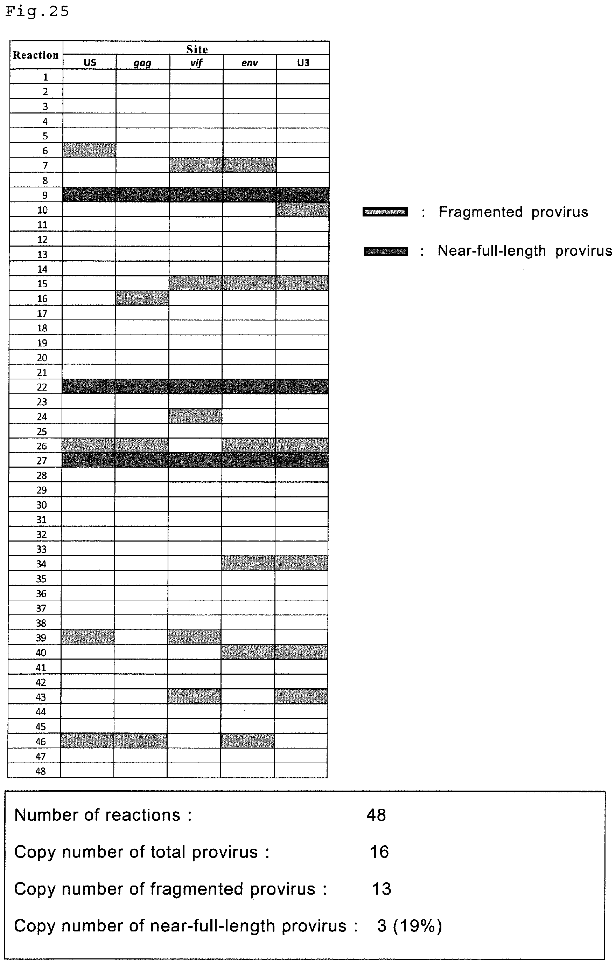

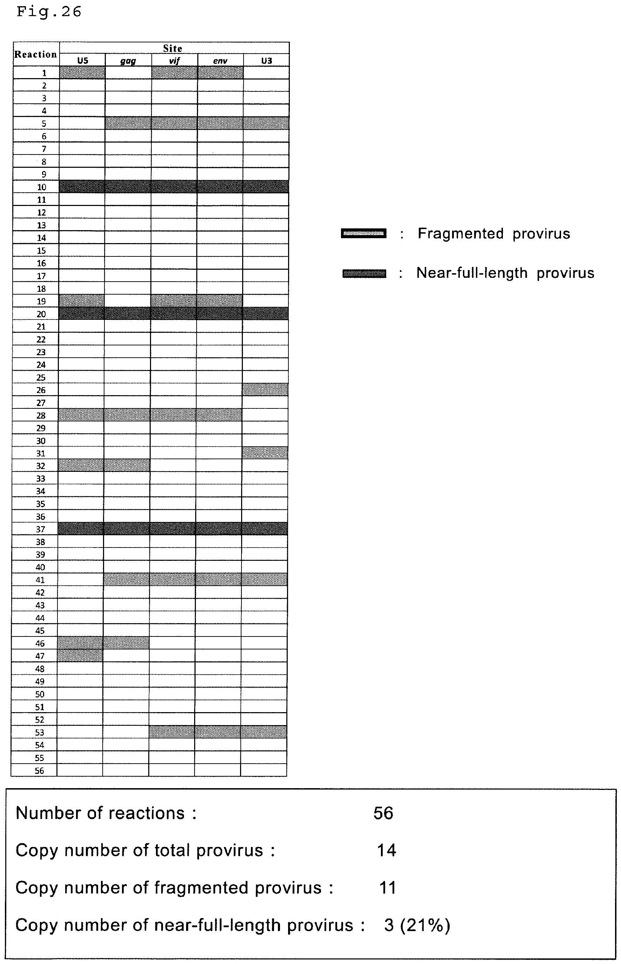

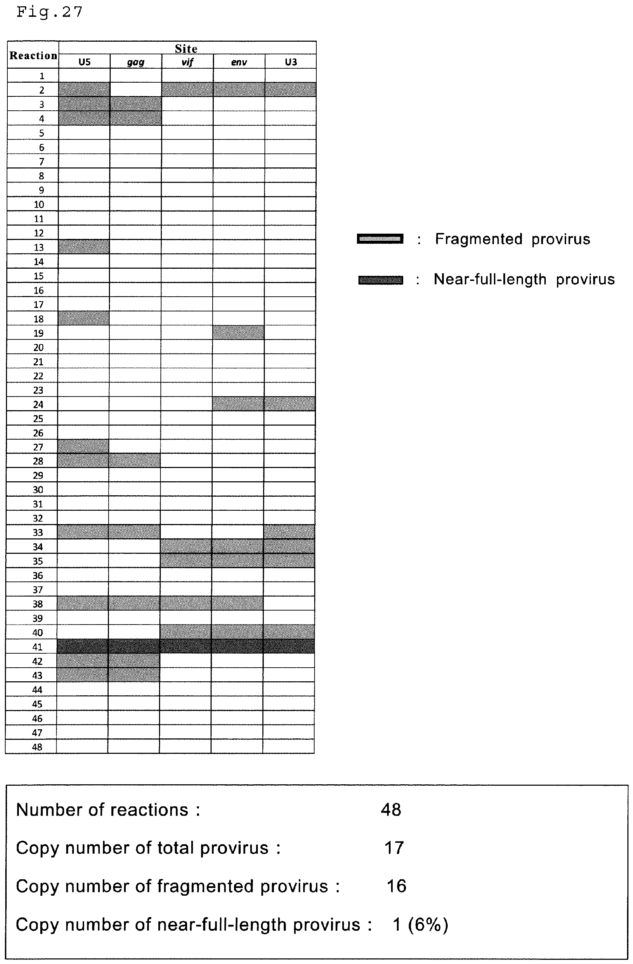

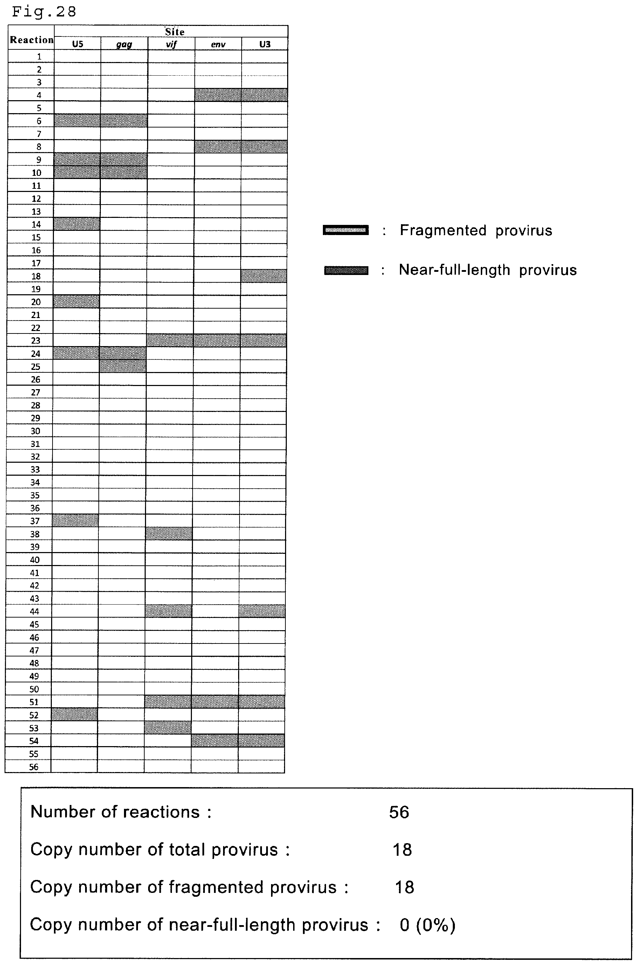

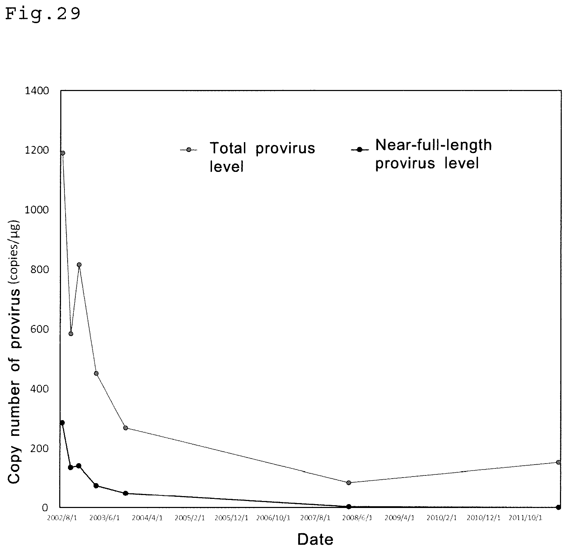

### (4) Follow-up study for 9 years after start of anti-HIV therapy (Figs. 22 to 29)

As years passed after the start of anti-HIV therapy, both the level of total provirus including defective provirus and the level of near-full-length provirus decreased. However, in those samples which underwent more than about 5 years of anti-HIV therapy, near-full-length provirus was not detected although the total provirus count was 80-150 copies/µg DNA. These results shows that the level of near-full-length provirus reflects the effect of anti-HIV therapy more accurately than the level of total provirus.

### Discussion

It has been confirmed that one copy of HIV-1 provirus can be quantified at 5 sites (U5, *gag, vif, env* and U3) using the present invention. Further, it has been suggested that provirus in infected individuals where it is capable of amplification undergoes fragmentation while viral replication is inhibited for a prolonged period by anti-HIV therapy. Since quantification of near-full-length provirus can be performed by PCR alone without requiring culture operations, such quantification is believed to be a promising method for monitoring the reservoir sizes in a large number of patients receiving anti-HIV therapy.

**Table 1. Nucleotide Sequences of Primers used in the Detection of Near-Full-Length Provirus**

| First-Round PCR | |
|---|---|
| U5-1F | ATCTGAGCCTGGGAGCTCTC |
| U5-1R | TAATACYGACGCTCTCGCMCC |
| gag-1F | CACTAGAAGAAATGATGACAGCATG |
| gag-1R | AAGCTCTCYKCTGGTGGRG |
| vif-1F | AACAGATGGCAGGTGATGATTG |
| vif-1R | TTTCCTCCATTCTAYGGAGRCTCC |
| env-1F | ATGAGGGAYAATTGGAGAAGTGA |
| env-1R | CTGCGCCCATAGTGCTTCCT |
| U3-1F | GCAGTAGSTGAGGGRACAGATAG |
| U3-1R | TGATCCCTGGCCCTGKTGTGTA |
| | |

| Second-Round PCR | |
|---|---|
| U5-2F | GCTARCTAGGGAACCCACTGC |
| U5-2R | GGGCGCCACTGCTAGAGATT |
| gag-2F | ARGGGCTGTTGGAAATGTGG |
| gag-2R | TTCCCTAAAAAATTAGCCTGYCT |
| vif-2F | GTGGCAAGTRGACAGGATGAGG |
| vif-2R | CCTGTATGCAGACCCCAATATGT |
| env-2F | AATTGAACCATTAGGANTAGCACC |
| env-2R | CTTCCTGCTGCTCCCAAGAACC |
| U3-2F | CCTCAGGTACCTTTAAGRCCAATGA |
| U3-2R | GGAGTAAATTADCCCTTCCAGTCC |

**Table 2. Composition of Reagents used in the Detection of Near-Full-Length Provirus**

| First-Round Multiplex PCR | |
|---|---|
| Reagent | For one reaction |
| 10 × PCR Buffer (Platinum) | 5.0 µl |
| 50 mM MgCl₂ | 3.0 µl |
| 25 mM each dNTP | 0.4 µl |
| 50 µM U5-1F | 0.2 µl |
| 50 µM U5-1R | 0.2 µl |
| 50 µM p7-1F | 0.2 µl |
| 50 µM p7-1R | 0.2 µl |
| 50 µM vif-1F | 0.2 µl |
| 50 µM vif-1R | 0.2 µl |
| 50 µM env-1F | 0.2 µl |
| 50 µM env-1R | 0.2 µl |
| 50 µM U3-1F | 0.2 µl |
| 50 µM U3-1R | 0.2 µl |
| Water | 19.4 µl |
| Platinum Taq | 0.2 µl |
| Total | 30.0 µl |

| | |
|---|---|
| Second-Round Real-Time PCR | |
| Reagent | For one reaction |
| 10 × PCR Buffer (Platinum) | 2.00 µl |
| 50 mM MgCl₂ | 1.20 µl |
| 25 mM each dNTP | 0.16 µl |
| 50 µM primer-2F | 0.08 µl |
| 50 µM primer-2R | 0.08 µl |
| 25 µM ROX Dye | 0.40 µl |
| 1/1,000x SYBR Green | 1.00 µl |
| Water | 10.00 µl |
| Platinum Taq | 0.08 µl |
| Total | 15.00 µl |

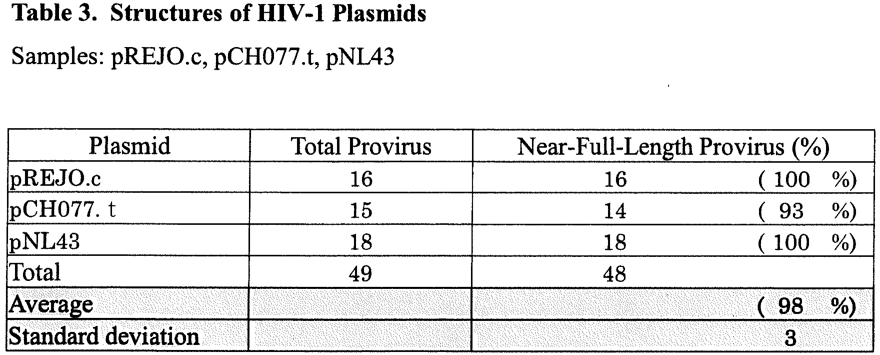

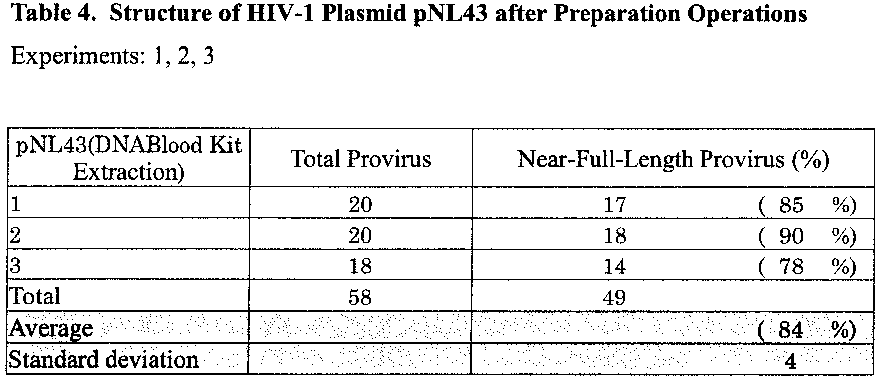

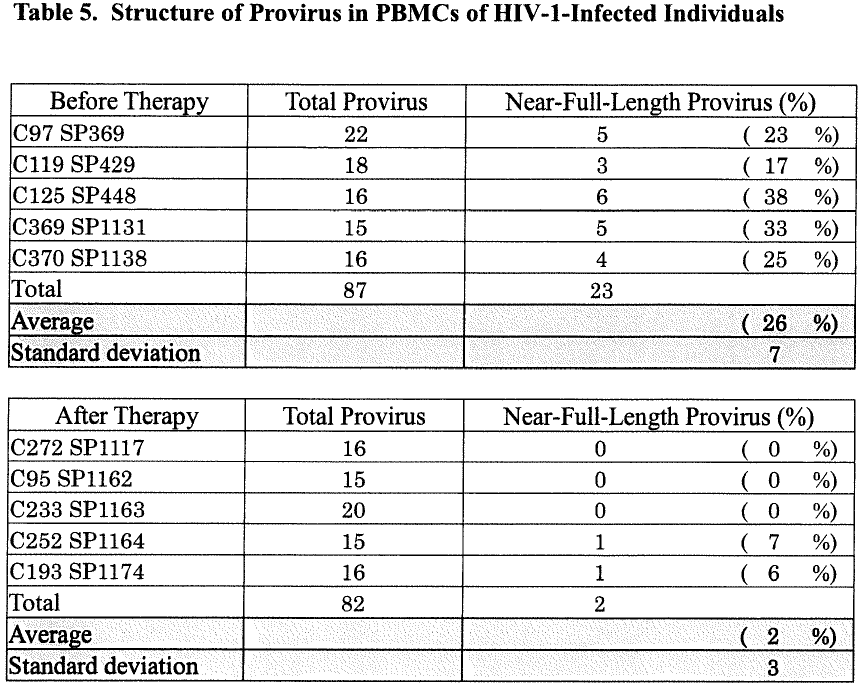

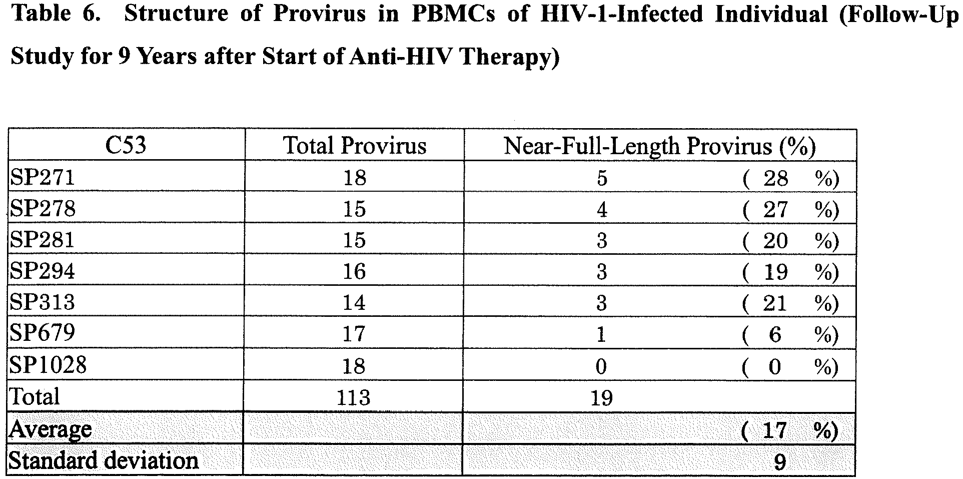

### [Example 2]

### Experimental Methods

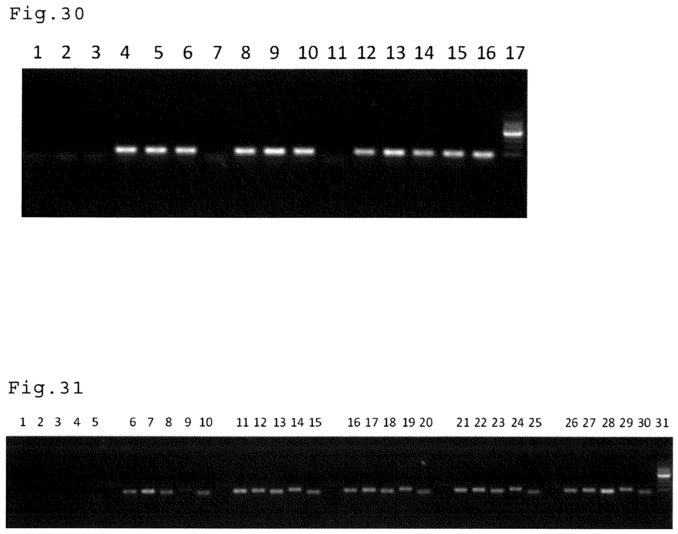

### (1) Multiplex/nested PCR (Fig. 30)

Multiplex/nested PCR products were subjected to agarose gel electrophoresis. Specifically, electrophoresis was carried out using 1x TAE buffer and 2.0% agarose gel under a voltage of 8 V/cm for 15 min. The first-round PCR was performed in a 25 µl solution containing 1x PCR buffer (Invitrogen), 4 mM of MgCl₂, 0.2 mM each of dNTPs, 0.12 µM each of the primers (U5-1FG, U5-1RG, gag-1FG, gag-1RG, vif-1FG, vif-1RG env-1FG, env-1RG, U3-1FG and U3-1RG), 0.1 µl of Platinum Taq DNA polymerase (Invitrogen) and 50 ng of human cellular DNA. The second-round PCR was performed in a 25 µl solution containing 1x PCR buffer, 4 mM of MgCl₂, 0.2 mM each of dNTPs, 0.12 µM each of the primers (U5-2FG, U5-2RG, gag-2FG, gag-2RG, vif-2FG, vif-2RG, env-2FG, env-2RG, U3-2FG and U3-2RG), 0.1 µl of Platinum Taq DNA polymerase (Invitrogen) and 1 µl of the first-round PCR product. Thermal conditions of the first-round PCR were as follows: reaction at 97°C for 2 min; 5 cycles of reaction at 97°C for 5 s, at 58°C for 10 s and at 72°C for 15 s; 35 cycles of reaction at 94°C for 5 s, at 58°C for 10 s and at 72°C for 15 s; and finally, cooling at 4°C. Thermal conditions of the second-round PCR were as follows: reaction at 94°C for 2 min; 30 cycles of reaction at 94°C for 5 s, at 58°C for 10 s and at 72°C for 15 s; and finally, cooling at 4°C. Lane 1 was negative control, where TE was added to the PCR. In lanes 2 to 15, an average of one copy of Not I-restricted HIV-1 DNA plasmid pTRJO.c was added per reaction. Lane 16 was positive control, where 10 copies of Not I-restricted HIV-1 DNA plasmid pRHPA.c were added. Lane 17 is a 100 bp DNA ladder (Takara Bio).

### PCR Primers

- Nucleotide sequence of upstream primer U5-1FG for amplifying a U5 region in the first-round PCR:
   5'-GGCTAACTAGGGAACCCACTGC-3' (SEQ ID NO: 21)
   (Number of nucleotides: 22; Nucleotide numbers in standard clone HXB2: 496-517)
- Nucleotide sequence of downstream primer US-1RG for amplifying a U5 region in the first-round PCR:
   5'-AGCAAGCCGAGTCCTGCG-3' (SEQ ID NO: 22)
   (Number of nucleotides: 18; Nucleotide numbers in standard clone HXB2: 707-690)
- Nucleotide sequence of upstream primer gag-1FG for amplifying a *gag* region in the first-round PCR:
   5'-GGGACATCAAGCAGCYATGC-3' (SEQ ID NO: 23)
   (Number of nucleotides: 20; Nucleotide numbers in standard clone HXB2: 1365-1384)
- Nucleotide sequence of downstream primer gag-1RG for amplifying a *gag* region in the first-round PCR:
   5'-ATCCATCCTATTTGTTCCTGAAGGKTACT-3' (SEQ ID NO: 24)
   (Number of nucleotides: 29; Nucleotide numbers in standard clone HXB2: 1538-1510)
- Nucleotide sequence of upstream primer vif-1F for amplifying a *vif* region in the first-round PCR:
   5'-CCTAGGTGTGAHTATCMAGCAGGACA-3' (SEQ ID NO: 25)
   (Number of nucleotides: 26; Nucleotide numbers in standard clone HXB2: 5431-5456)
- Nucleotide sequence of downstream primer vif-1RG for amplifying a *vif* region in the first-round PCR:
   5'-GTCTTCKGGGGCTTGTTCCATCT-3' (SEQ ID NO: 26)
   (Number of nucleotides: 23; Nucleotide numbers in standard clone HXB2: 5579-5557)
- Nucleotide sequence of upstream primer env-1FG for amplifying an *env* region in the first-round PCR:
   5'-GAGATATGAGGGAYAATTGGAGAAGTGA-3' (SEQ ID NO: 27)
   (Number of nucleotides: 28; Nucleotide numbers in standard clone HXB2: 7642-7669)
- Nucleotide sequence of downstream primer env-1RG for amplifying an *env* region in the first-round PCR:
   5'-GCGCCCATAGTGCTTCCTGC-3' (SEQ ID NO: 28)
   (Number of nucleotides: 20; Nucleotide numbers in standard clone HXB2: 7819-7800)
- Nucleotide sequence of upstream primer U3-1FG for amplifying a U3 region in the first-round PCR:
   5'-GTGGGTTTTCCAGTCARRCCTCA-3' (SEQ ID NO: 29)
   (Number of nucleotides: 23; Nucleotide numbers in standard clone HXB2: 8992-9014)
- Nucleotide sequence of downstream primer U3-1RG for amplifying a U3 region in the first-round PCR:
   5'-RYCCCTGGCCCTGGTGTGTA-3' (SEQ ID NO: 30)
   (Number of nucleotides: 20; Nucleotide numbers in standard clone HXB2: 9194-9175)
- Nucleotide sequence of upstream primer U5-2FG for amplifying a U5 region in the second-round PCR:
   5'-GCTTCAAGTAGTGTGTGCCCGT-3' (SEQ ID NO: 31)
   (Number of nucleotides: 22; Nucleotide numbers in standard clone HXB2: 546-567)
- Nucleotide sequence of downstream primer U5-2RG for amplifying a U5 region in the second-round PCR:
   5'-GGGCGCCACTGCTAGAGATTTT-3' (SEQ ID NO: 32)
   (Number of nucleotides: 22; Nucleotide numbers in standard clone HXB02: 643-622)
- Nucleotide sequence of upstream primer gag-2FG for amplifying a *gag* region in the second-round PCR:
   5'-CAATGAGGAAGCTGCAGAATGGGATA-3' (SEQ ID NO: 33)
   (Number of nucleotides: 26; Nucleotide numbers in standard clone HXB2: 1404-1429)
- Nucleotide sequence of downstream primer gag-2RG for amplifying a *gag* region in the second-round PCR:
   5'-TAGTTCCTGCTATGTCACTTCCCCT-3' (SEQ ID NO: 34)
   (Number of nucleotides: 25; Nucleotide numbers in standard clone HXB2: 1507-1483)
- Nucleotide sequence of upstream primer vif-2FG for amplifying a *vif* region in the second-round PCR:
   5'-CAAGGTAGGATCYCTACARTACTTGGC-3' (SEQ ID NO: 35)
   (Number of nucleotides: 27; Nucleotide numbers in standard clone HXB2: 5460-5486)
- Nucleotide sequence of downstream primer vif-2RG for amplifying a *vif* region in the second-round PCR:
   5'-AGTTTCBYAACACTAGGYAAAGGTGG-3' (SEQ ID NO: 36)
   (Number of nucleotides: 26; Nucleotide numbers in standard clone HXB2: 5546-5521)
- Nucleotide sequence of upstream primer env-2FG for amplifying an *env* region in the second-round PCR:
   5'-GTAAAAATTGAACCATTAGGRRTAGCACC-3' (SEQ ID NO: 37)
   (Number of nucleotides: 29; Nucleotide numbers in standard clone HXB2: 7689-7717)
- Nucleotide sequence of downstream primer env-2RG for amplifying an *env* region in the second-round PCR:
   5'-GCTTCCTGCTGCTCCCAARAA-3' (SEQ ID NO: 38)
   (Number of nucleotides: 21; Nucleotide numbers in standard clone HXB2: 7808-7788)
- Nucleotide sequence of upstream primer U3-2FG for amplifying a U3 region in the second-round PCR:
   5'-GACAAGATATCCTTGAYCTGTGGRT-3' (SEQ ID NO: 39)
   (Number of nucleotides: 25; Nucleotide numbers in standard clone HXB2: 9113?9137)
- Nucleotide sequence of downstream primer U3-2RG for amplifying a U3 region in the second-round PCR:
   5'-GTGTGTAGTTMTGCCARTCAGGGAA-3' (SEQ ID NO: 40)
   (Number of nucleotides: 25; Nucleotide numbers in standard clone HXB2: 9181?9157)
- "5'-" and "-3' " as indicated in the above nucleotide sequences represent 5' end and 3' end, respectively.

Symbols other than "A", "G", "C" and "T" appearing in the above nucleotide sequences represent the following.
R: A/G degeneracy
M: A/C degeneracy
K: T/G degeneracy
Y: T/C degeneracy
H: A/C/T degeneracy
B: G/C/T degeneracy

### (2) Individual Second-Round PCR (Fig. 31)

Individual second-round PCR products were subjected to agarose gel electrophoresis. Specifically, electrophoresis was carried out using 1x TAE buffer and 2.0% agarose gel under a voltage of 8 V/cm for 15 min. The individual second-round PCR was performed in a 25 µl solution containing 1x PCR buffer, 4 mM of MgCl₂, 0.2 mM each of dNTPs, 0.12 µM of any of the following primer pairs (U5-2FG/U5-2RG, gag-2FG/gag-2RG, vif-2FG/vif-2RG, env-2FG/env-2RG or U3-2FG/U3-2RG), 0.1 µl of Platinum Taq DNA polymerase (Invitrogen), and 1 µl of the first-round PCR product. Thermal conditions of the PCR were as follows: reaction at 94°C for 2 min; 30 cycles of reaction at 94°C for 5 s, at 58°C for 10 s and at 72°C for 15 s; and finally, cooling at 4°C. Lanes 1 to 5 were negative control, where first-round PCR product (1) (lane 1 in Fig. 1) was added to the PCR. In lanes 6 to 10, first-round PCR product (8) (lane 8 in Fig. 1) was added to the PCR. In lanes 11 to 15, first-round PCR product (10) (lane 10 in Fig. 1) was added to the PCR. In lanes 16 to 20, first-round PCR product (12) (lane 12 in Fig. 1) was added to the PCR. In lanes 21 to 25, first-round PCR product (15) (lane 15 in Fig. 1) was added to the PCR. Lanes 26 to 30 were positive control, where first-round PCR product (16) (lane 16 in Fig. 1) was added to the PCR. Lane 31 is a 100 bp DNA ladder (Takara Bio). In lanes 1, 6, 11, 16, 21 and 26, the primer pair U5-2FG/U5-2RG was used. In lanes 2, 7, 12, 17, 22 and 27, the primer pair gag-2FG/gag-2RG was used. In lanes 3, 8, 13, 18, 23 and 28, the primer pair vif-2FG/vif-2RG was used. In lanes 4, 9, 14, 19, 24 and 29, the primer pair env-2FG/env-2RG was used. In lanes 5, 10, 15, 20, 25 and 30, the primer pair U3-2FG/U3-2RG was used. Lane 31 is a 100 bp DNA ladder (Takara Bio).

### Results

Agarose gel electrophoresis of multiplex nested PCR products is shown in Fig. 30. Agarose gel electrophoresis of individual second-round PCR products is shown in Fig. 31.

In Fig. 30, no band appeared in the negative control lane 1, as expected. In the positive control lane 16, a band appeared at around 100 bp as expected in comparison with the molecular weight marker in lane 17. In ten out of 14 PCR reactions in lanes 2 to 15, a band similar to that from the positive control was detected at a ratio of 0.71. According to the Poisson distribution formula, when an average of one target DNA molecule is present per reaction, the ratio at which a positive reaction is detected is calculated as 0.63, which is in good agreement with the found value 0.71. Therefore, the results of this experiment demonstrate that one molecule of HIV-1 DNA can almost always be detected by the method of the present invention almost.

In Fig. 31, no band appeared in the negative control lanes 1 to 5, as expected. In the positive control lanes 26 to 30, bands with sizes in bp (compared to the molecular marker in lane 17) appeared as expected from the respective relevant primer pairs. In lanes 6 to 10, lanes 11 to 15, lanes 16 to 20 and lanes 21 to 25 which show the results of using the four first-round PCR products that were confirmed to be positive in Fig. 30, bands with sizes in bp as expected from the respective relevant primer pairs were detected in the same way as in the positive control lanes. These results demonstrate that, according to the method of the present invention, one molecule of full-length HIV-1 DNA is detected at 5 sites (U5, *gag, vif, env* and U3) simultaneously. This means that full-length HIV-1 DNA and fragmented HIV-1 DNA can be quantified as distinct entities.

### Discussion

These results reveal that, by using the multiplex nested PCR and individual second-round PCR as developed by the present inventor, a single copy of HIV-1 DNA molecule can be detected at 5 sites (U5, *gag, vif, env* and U3) simultaneously This means that, according to this technique, near-full-length HIV-1 provirus DNA can be quantified as a distinct entity from partial provirus DNA.

All publications, patents and patent applications cited herein are incorporated herein by reference in their entirety.

### INDUSTRIAL APPLICABILITY

The present invention is useful for predicting therapeutic effects on HIV-infected individuals and the progress of pathology in those individuals.

### SEQUENCE LISTING FREE TEXT

<SEQ ID NO: 1>
   SEQ ID NO: 1 shows the nucleotide sequence of forward primer U5-1F.
<SEQ ID NO: 2>
   SEQ ID NO: 2 shows the nucleotide sequence of reverse primer U5-1R.
<SEQ ID NO: 3>
   SEQ ID NO: 3 shows the nucleotide sequence of forward primer gag-1F.
<SEQ ID NO: 4>
   SEQ ID NO: 4 shows the nucleotide sequence of reverse primer gag-1R.
<SEQ ID NO: 5>
   SEQ ID NO: 5 shows the nucleotide sequence of forward primer vif-1F.
<SEQ ID NO: 6>
   SEQ ID NO: 6 shows the nucleotide sequence of reverse primer vif-1R.
<SEQ ID NO: 7>
   SEQ ID NO: 7 shows the nucleotide sequence of forward primer env-1F.
<SEQ ID NO: 8>
   SEQ ID NO: 8 shows the nucleotide sequence of reverse primer env-1R.
<SEQ ID NO: 9>
   SEQ ID NO: 9 shows the nucleotide sequence of forward primer U3-1F.
<SEQ ID NO: 10>
   SEQ ID NO: 10 shows the nucleotide sequence of reverse primer U3-1R.
<SEQ ID NO: 11>
   SEQ ID NO: 11 shows the nucleotide sequence of forward primer U5-2F.
<SEQ ID NO: 12>
   SEQ ID NO: 12 shows the nucleotide sequence of reverse primer U5-2R.
<SEQ ID NO: 13>
   SEQ ID NO: 13 shows the nucleotide sequence of forward primer gag-2F.
<SEQ ID NO: 14>
   SEQ ID NO: 14 shows the nucleotide sequence of reverse primer gag-2R.
<SEQ ID NO: 15>
   SEQ ID NO: 15 shows the nucleotide sequence of forward primer vif-2F.
<SEQ ID NO: 16>
   SEQ ID NO: 16 shows the nucleotide sequence of reverse primer vif-2R.
<SEQ ID NO: 17>
   SEQ ID NO: 17 shows the nucleotide sequence of forward primer env-2F.
<SEQ ID NO: 18>
   SEQ ID NO: 18 shows the nucleotide sequence of reverse primer env-2R.
<SEQ ID NO: 19>
   SEQ ID NO: 19 shows the nucleotide sequence of forward primer U3-2F.
<SEQ ID NO: 20>
   SEQ ID NO: 20 shows the nucleotide sequence of reverse primer U3-2R.
SEQ ID NO: 21>
   SEQ ID NO: 21 shows the nucleotide sequence of forward primer U5-1FG.
<SEQ ID NO: 22>
   SEQ ID NO: 22 shows the nucleotide sequence of reverse primer U5-1RG.
<SEQ ID NO: 23>
   SEQ ID NO: 23 shows the nucleotide sequence of forward primer gag-1FG.
<SEQ ID NO: 24>
   SEQ ID NO: 24 shows the nucleotide sequence of reverse primer gag-1RG.
<SEQ ID NO: 25>
   SEQ ID NO: 25 shows the nucleotide sequence of forward primer vif-1FG.
<SEQ ID NO: 26>
   SEQ ID NO: 26 shows the nucleotide sequence of reverse primer vif-1RG.
<SEQ ID NO: 27>
   SEQ ID NO: 27 shows the nucleotide sequence of forward primer env-1FG.
<SEQ ID NO: 28>
   SEQ ID NO: 28 shows the nucleotide sequence of reverse primer env-1RG.
<SEQ ID NO: 29>
   SEQ ID NO: 29 shows the nucleotide sequence of forward primer U3-1FG.
<SEQ ID NO: 30>
   SEQ ID NO: 30 shows the nucleotide sequence of reverse primer U3-1RG.
<SEQ ID NO: 31>
   SEQ ID NO: 31 shows the nucleotide sequence of forward primer U5-2FG.
<SEQ ID NO: 32>
   SEQ ID NO: 32 shows the nucleotide sequence of reverse primer U5-2RG.
<SEQ ID NO: 33>
   SEQ ID NO: 33 shows the nucleotide sequence of forward primer gag-2FG.
<SEQ ID NO: 34>
   SEQ ID NO: 34 shows the nucleotide sequence of reverse primer gag-2RG.
<SEQ ID NO: 35>
   SEQ ID NO: 35 shows the nucleotide sequence of forward primer vif-2FG.
<SEQ ID NO: 36>
   SEQ ID NO: 36 shows the nucleotide sequence of reverse primer vif-2RG.
<SEQ ID NO: 37>
   SEQ ID NO: 37 shows the nucleotide sequence of forward primer env-2FG.
<SEQ ID NO: 38>
   SEQ ID NO: 38 shows the nucleotide sequence of reverse primer env-2RG.
<SEQ ID NO: 39>
   SEQ ID NO: 39 shows the nucleotide sequence of forward primer U3-2FG.
<SEQ ID NO: 40>
   SEQ ID NO: 40 shows the nucleotide sequence of reverse primer U3-2RG.

## Claims

1. A marker showing HIV pathology, comprising near-full-length HIV provirus.

2. A method of testing HIV pathology, comprising evaluating the level of near-full-length HIV provirus in HIV-infected cells.

3. The method of claim 2, wherein it is judged that a functional cure of HIV has been achieved when near-full-length HIV provirus in infected cells is evaluated as below a specific detection limit, and it is judged that a functional cure of HIV has not been achieved when near-full-length HIV provirus in infected cells is evaluated as equal to or above the specific detection limit.

4. The method of claim 2 or 3, wherein the level of near-full-length HIV provirus in HIV-infected cells is evaluated by detecting 3 to 7 regions scattered across the full length of HIV provirus.

5. A method of testing the efficacy of an anti-HIV drug, comprising evaluating the level of near-full-length HIV provirus in HIV-infected cells.

6. The method of claim 5, wherein the level of near-full-length HIV provirus in HIV-infected cells is evaluated before and after anti-HIV drug administration and/or before and after a specific period during continuation of anti-HIV drug administration, and it is judged that a functional cure of HIV has been achieved or is being achieved when the copy number of near-full-length HIV provirus has decreased, and it is judged that the anti-HIV drug does not enable a functional cure of HIV when the copy number of near-full-length HIV provirus has not decreased.

7. A method of screening for drugs that enable a functional cure of HIV, comprising evaluating the level of near-full-length HIV provirus in HIV-infected cells.

8. The method of claim 7, wherein the level of near-full-length HIV provirus in HIV-infected cells is evaluated before and after administration of a drug and/or before and after a specific period during continuation of administration of the drug, and it is judged that the drug will enable a functional cure of HIV when the copy number of near-full-length HIV provirus has decreased, and it is judged that the drug does not enable a functional cure of HIV when the copy number of near-full-length HIV provirus has not decreased.

9. A method of evaluating the level of near-full-length HIV provirus in HIV-infected cells, comprising detecting 3 to 7 regions scattered across the full length of HIV provirus.

10. The method of any one of claims 1 to 9, comprising evaluating the level of near-full-length HIV provirus in HIV-infected cells using primers of 20 to 30 nucleotides comprising a part or the whole of any nucleotide sequence as shown in SEQ ID NOS: 1 to 40.

11. A kit for evaluating the level of near-full-length HIV provirus, comprising primers capable of amplifying 3 to 7 regions scattered across the full length of HIV provirus and reagents capable of detecting the nucleic acid amplified by the primers.

12. The kit of claim 11, wherein the primers capable of amplifying 3 to 7 regions scattered across the full length of HIV provirus are primers of 20 to 30 nucleotides comprising a part or the whole of any nucleotide sequence as shown in SEQ ID NOS: 1 to 40.
